# EUROPÄISCHE PATENTSCHRIFT

(11) **EP 3 498 326 B1**
(45) Veröffentlichungstag und Bekanntmachung des Hinweises auf die Patenterteilung: **29.11.2023**
(21) Anmeldenummer: 17208068.1
(22) Anmeldetag: 18.12.2017
(51) Int. Cl.: A61M 15/00, A61M 11/00, B05B 11/00, G06M 3/02

(54) **TEILZÄHLER FÜR EINEN ZERSTÄUBER, ZERSTÄUBER MIT EINEM SOLCHEN TEILZÄHLER, UND SYSTEM MIT EINEM SOLCHEN ZERSTÄUBER UND EINER DOCKINGSTATION**
PART COUNTER FOR A NEBULISER, NEBULISER WITH SUCH A PART COUNTER, AND SYSTEM WITH SUCH A NEBULISER AND A DOCKING STATION
COMPTEUR PARTIEL POUR NÉBULISATEUR, NÉBULISATEUR COMPRENANT UN TEL COMPTEUR PARTIEL, ET SYSTÈME COMPRENANT UN TEL NÉBULISATEUR ET UNE STATION D'ACCUEIL

(43) Veröffentlichungstag der Anmeldung: 19.06.2019
(73) Patentinhaber: Boehringer Ingelheim International GmbH, 55216 Ingelheim am Rhein (DE)
(72) Erfinder: EICHER, Joachim, Ingelheim am Rhein (DE); JUNG, Andree, Ingelheim am Rhein (DE); WUTTKE, Gilbert, Ingelheim am Rhein (DE)
(74) Vertreter: Von Rohr Patentanwälte Partnerschaft mbB

(56) Entgegenhaltungen:
- WO-A1-00/64517
- WO-A1-2005/080001
- WO-A1-2011/157561
- WO-A1-2015/169430
- WO-A2-03/020349
- WO-A2-2007/022898
- US-A1- 2003 178 020
- US-A1- 2011 253 139
- US-A1- 2017 035 976

## Beschreibung

Die vorliegende Erfindung betrifft einen Teilzähler nach dem Oberbegriff des Anspruchs 1, einen Zerstäuber mit einem solchen Teilzähler, und ein System mit einem solchen Zerstäuber und einer Dockingstation.

Ein "Zerstäuber" im Sinne der vorliegenden Erfindung ist vorzugsweise dazu ausgebildet, eine Substanz, beispielsweise ein Pulver oder ein Fluid, insbesondere eine Arzneimittelformulierung, zu zerstäuben bzw. in ein Aerosol zu überführen. Das erzeugte Aerosol kann dann von einem Benutzer bzw. Patienten eingeatmet bzw. inhaliert werden, insbesondere zur Behandlung einer Krankheit. Ein Zerstäuber ist vorzugsweise ein portables bzw. mobiles Gerät.

Ein Zerstäuber im Sinne der vorliegenden Erfindung weist vorzugsweise einen Behälter als Reservoir für die zu zerstäubende Substanz auf. Der Behälter ist vorzuasweise zur Abgabe der Substanz, insbesondere des Fluids, relativ zu einem Gehäuseteil - vorzugsweise axial - bewegbar.

Besonders bevorzugt weist ein Zerstäuber im Sinne der vorliegenden Erfindung einen mechanischen Energiespeicher auf, dem Energie hinzufügbar ist. Vorzugsweise ist der Energiespeicher als spannbare bzw. komprimierbare (Antriebs-)Feder ausgebildet, wobei durch Entspannung der Feder die zu zerstäubende Substanz unter Druck gesetzt und über eine Düse bzw. Austragsdüse zerstäubt bzw. als einatembares Aerosol ausgegeben wird. Ein solcher Zerstäuber ist beispielsweise aus der WO 2009/047173 bekannt.

Ferner ist in der WO 2005/080001 eine Überwachungseinrichtung zur Zählung von Betätigungen des Zerstäubers offenbart, wobei der Zählwert der Betätigungen des Zerstäubers manuell oder selbsttätig rücksetzbar ist.

Die WO 2007/022898 offenbart einen Zerstäuber mit zwei Zählern, wobei der erste Zähler die Benutzungen des Zerstäubers zählt und der zweite Zähler die in den Zerstäuber eingesetzten oder mit dem Zerstäuber benutzten Behälter zählt.

Die WO 2015/169430 A1 offenbart einen Zähler zum Zählen von Benutzungen eines Bestäubers, wobei der Zähler zurücksetzbar ist.

Die WO 2011/157561 A1 offenbart einen Zähler zum Zählen von Benutzungen eines Zerstäubers, wobei der Zähler mit dem Zerstäuber koppelbar und von dem Zerstäuber abnehmbar ist.

Die US 2003/0178020 A1 offenbart einen Zähler zum Zählen von Benutzungen eines Zerstäubers, wobei der Zähler mit dem Zerstäuber koppelbar ist.

Die US 2017/0035976 A1 offenbart einen Zähler zum Zählen von Benutzungen eines Zerstäubers, wobei der Zähler mit dem Zerstäuber koppelbar ist.

Die WO 00/64517 A1 offenbart einen Zähler zum Zählen von Benutzungen eines Zerstäubers, wobei der Zähler mit dem Zerstäuber koppelbar und von dem Zerstäuber abnehmbar ist.

Die US 2011/0253139 A1 offenbart einen Zähler zum Zählen von Benutzungen eines Zerstäubers, wobei der Zähler mit dem Zerstäuber koppelbar und von dem Zerstäuber abnehmbar ist.

Gattungsgemäße Zerstäuber werden insbesondere bei langfristigen Behandlungsverläufen oftmals über den Tag verteilt mehrfach benutzt, um eine quasi-kontinuierliche Zufuhr von Wirkstoff in der benötigten Dosierung zu ermöglichen. Werden hierbei einzelne Benutzungen vergessen, entsteht eine Unterdosierung, während eine Überdosierung dadurch hervorgerufen werden kann, dass versehentlich zu viele Benutzungen erfolgen. Derartige Dosierungsfehler können zu schwerwiegenden Folgen führen.

Eine Aufgabe der vorliegenden Erfindung ist es daher, einen Teilzähler, einen Zerstäuber und ein System anzugeben, wodurch die Anwendungssicherheit bei einer Benutzung des Zerstäubers verbessert werden kann.

Die obige Aufgabe wird durch einen Teilzähler gemäß Anspruch 1, einen Zerstäuber gemäß Anspruch 10 oder ein System gemäß Anspruch 14 gelöst. Vorteilhafte Weiterbildungen sind Gegenstand der Unteransprüche.

### ZUSAMMENFASSUNG DER ERFINDUNG

Die vorliegende Erfindung bezieht sich auf einen Teilzähler gemäß Anspruch 1, einen Zerstäuber gemäß Anspruch 10 und ein System gemäß Anspruch 14. Vorteilhafte Weiterbildungen der Erfindung sind Gegenstand der Unteransprüche.

### ZUSAMMENFASSUNG DER VORLIEGENDEN OFFENBARUNG

Ein vorschlagsgemäßer Teilzähler ist zum Zählen von Benutzungen eines Zerstäubers ausgebildet, vorzugsweise wobei der Zerstäuber zur Erzeugung eines einatembaren Aerosols ausgebildet ist. Insbesondere ist der Teilzähler dazu ausgebildet, einen Teil der Benutzungen des Zerstäubers zu zählen. Vorzugsweise ist der Teilzähler dazu ausgebildet, bei jeder Erzeugung (einer Dosis) des Aerosols (genau) eine Benutzung zu zählen bzw. einen Zählerwert oder Zählerstand genau um den Wert 1 zu erhöhen.

Unter dem Begriff "Aerosol" ist bei der vorliegenden Offenbarung vorzugsweise eine wolkenartige oder nebelartige Ansammlung einer Vielzahl von flüssigen oder festen Schwebeteilchen einer mittels eines Zerstäubers zerstäubten Substanz zu verstehen, vorzugsweise wobei die Schwebeteilchen eine geringe Geschwindigkeit und/oder zumindest im Wesentlichen ungerichtete Bewegungsrichtungen aufweisen. Ein "Aerosol" kann beispielsweise eine kegelförmige Teilchenwolke aufweisen oder bilden, insbesondere wobei die Hauptausbreitungsrichtung der Teilchenwolke zumindest im Wesentlichen der Hauptaustrittsrichtung bzw. Austrittsimpulsrichtung entspricht.

Bei der Zerstäubung einer Substanz bzw. eines Fluids, insbesondere einer flüssigen Arzneimittelformulierung, mittels eines Zerstäubers der eingangs erwähnten Art soll eine möglichst genau definierte Menge an Wirkstoff in ein Aerosol zur Inhalation überführt werden. Das auf diese Weise erzeugte Aerosol soll sich durch einen kleinen Mittelwert der Tropfengröße bei einer schmalen Tropfengrößenverteilung und einem niedrigen Impuls bzw. eine niedrige Ausbreitungsgeschwindigkeit auszeichnen. Vorzugsweise weisen die so erzeugten Tropfen einen Tropfendurchmesser von weniger als 5 Mikrometer, insbesondere zwischen 2 Mikrometer und 5 Mikrometer, auf, da Tropfen dieser Größe bei der Inhalation in geeigneter Weise in Lungensystemen abgeschieden werden.

Der Begriff "Fluid" ist im Sinne der vorliegenden Offenbarung breit zu verstehen und auszulegen. Vorzugsweise handelt es sich bei dem Fluid um eine Flüssigkeit. Insbesondere umfasst der Begriff "Fluid" bzw. "Flüssigkeit" auch Dispersionen, Suspensionen o. dgl.

Ein vorschlagsgemäßer Teilzähler ist mit einem Zerstäuber - vorzugsweise mechanisch - koppelbar bzw. verbindbar und von dem Zerstäuber abnehmbar bzw. trennbar. Insbesondere ist der Teilzähler als Add-On-Device, Zubehörteil und/oder Nachrüstteil ausgebildet. Dies ermöglicht auf einfache und kostengünstige Weise das Nachrüsten eines Zerstäubers mit einem (Teil-)Zähler.

Der Teilzähler ist vorzugsweise separat oder separierbar von einem Gehäuse des Zerstäubers gebildet. Hierdurch ist der Teilzähler vorzugsweise nachträglich anbringbar, austauschbar und/oder wiederverwendbar. Insbesondere kann ein bereits vorhandener Zerstäuber mit dem Teilzähler nachgerüstet werden, wodurch ein vorschlagsgemäßer Zerstäuber gebildet werden kann. Hierbei ist der Teilzähler vorzugsweise dazu ausgebildet, bei oder durch Anbringung an einem Gehäuse des Zerstäubers mit dem Zerstäuber derart gekoppelt zu werden oder koppelbar zu sein, dass der Teilzähler Benutzungen des Zerstäubers (automatisch) detektieren kann. Diese Detektion kann beispielsweise mechanisch, optisch und/oder elektronisch erfolgen.

Gemäß einem Aspekt der vorliegenden Offenbarung weist der Teilzähler eine Rücksetzeinrichtung zum Zurücksetzen des Teilzählers auf. Ebenso ist es bevorzugt, dass der Teilzähler eine vorzugsweise mechanische Detektionseinrichtung zum Detektieren von Benutzungen des Zerstäubers aufweist.

Eine "Benutzung" des Zerstäubers im Sinne der vorliegenden Offenbarung ist vorzugsweise eine Erzeugung (genau) einer Dosis eines Aerosols mit dem Zerstäuber und/oder eine Einnahme (genau) einer Dosis des erzeugten Aerosols durch einen Benutzer bzw. Patienten.

Vorzugsweise wird zum Zählen oder Erfassen einer Benutzung des Zerstäubers ein Schritt erfasst oder gezählt, der zur Erzeugung (genau) einer Dosis eines Aerosols mit dem Zerstäuber und/oder zur Einnahme (genau) einer Dosis des erzeugten Aerosols erforderlich ist, detektiert, insbesondere mittels einer Detektionseinrichtung.

Üblicherweise umfassen die Erzeugung des Aerosols und die Einnahme des Aerosols mehrere Schritte, die zwar nacheinander bzw. in einer bestimmten Reihenfolge, aber nicht zwingend zeitlich unmittelbar nacheinander ausgeführt werden (müssen). Beispielweise kann eine Erzeugung des Aerosols zunächst vorbereitet werden und die tatsächliche Erzeugung des Aerosols dann aber erst zu einem beliebigen späteren Zeitpunkt ausgelöst werden.

Verschiedene im Folgenden erläuterte Schritte, die (nacheinander) zur Erzeugung derselben Aerosol-Dosis durchgeführt werden bzw. erforderlich sind, werden jedoch vorzugsweise nur als (genau) eine Benutzung des Zerstäubers gezählt bzw. gewertet. Mehrere (aufeinander folgende) Schritte zur Erzeugung und/oder Einatmung derselben Aerosol-Dosis werden also vorzugsweise nicht als verschiedene Benutzungen des Zerstäubers gezählt bzw. gewertet, auch wenn es möglich ist, dass mehrere der Schritte detektiert bzw. erfasst werden.

Vorzugsweise korrespondiert also die Erzeugung genau einer Dosis des Aerosols zu genau einer (gezählten) Benutzung des Zerstäubers und umgekehrt.

Insbesondere können einer oder mehrere der folgenden Schritte zur Erzeugung und/oder Einnahme des Aerosols bzw. (genau) einer Dosis des Aerosols erforderlich sein und/oder zum Zählen einer Benutzung des Zerstäubers detektiert werden: ein Zerstäuben der Substanz bzw. des Fluids eine Erzeugung und/oder ein Austrag des Aerosols, eine Handhabung des Zerstäubers zur Vorbereitung einer Erzeugung des Aerosols wie insbesondere das Beladen einer Dosierkammer mit auszubringender bzw. zu zerstäubender Substanz, das Einbringen von Energie in einen Energiespeicher des Zerstäubers beispielsweise durch Spannen einer Antriebsfeder des Zerstäubers (auch als "Ladevorgang" des Energiespeichers bezeichnet) oder ein Verdrehen von Gehäuseteilen gegeneinander zum Spannen einer Antriebsfeder, eine Betätigung eines Auslösers bzw. eine Auslösung des Zerstäubers, und/oder ein Einatmen bzw. Inhalieren des erzeugten Aerosols.

Eine "Benutzung" ist also vorzugsweise der Aerosolerzeugung genau einer einzelnen Dosis der Substanz zugeordnet bzw. entspricht vorzugsweise genau einem kontinuierlichen Austrag der abzugebenden Substanz. Ursächlich hierfür kann genau eine Auslösung oder genau eine Aerosolbildung oder genau eine sonstige Betätigung des Zerstäubers sein.

Eine Detektion einer Benutzung kann unmittelbar oder mittelbar erfolgen, insbesondere mittelbar durch eine Detektion eines Hinzufügens von Energie zu dem mechanischen Energiespeicher bzw. eines Spannens, insbesondere Komprimierens, der Feder und/oder mittelbar durch eine Detektion einer Betätigung eines Auslösers bzw. einer Auslösung des Zerstäubers. Eine unmittelbare Detektion kann beispielsweise eine Detektion des erzeugten Aerosols, eine Detektion des Austrags des Aerosols oder eine Detektion eines Einatem- bzw. Inhalationsvorgangs sein.

Mittels der Rücksetzeinrichtung ist es möglich, in regelmäßigen Abständen - beispielsweise jeden Tag bzw. Abend - den Teilzähler zurückzusetzen, so dass ein Benutzer den Teilzähler beispielsweise dazu verwenden kann, die täglich erfolgten Benutzungen zu ermitteln, zu erfassen und/oder zu zählen. Der Teilzähler kann also insbesondere als Tagesdosiszähler dienen bzw. als Tagesdosiszähler ausgebildet sein.

Als "Tagesdosiszähler" wird hierbei vorzugsweise ein Zähler bzw. Teilzähler bezeichnet, dessen Wert einer innerhalb eines bestimmten oder vorbestimmbaren Zeitraums, insbesondere eines Tages, durch den Zerstäuber ausgegebenen Gesamtdosis bzw. einer Anzahl von innerhalb eines Tages erfolgten Betätigungen entspricht. Bei dem Tagesdosiszähler handelt es sich also vorzugsweise um eine Einrichtung, die (nur oder ausschließlich) die Anzahl von Benutzungen innerhalb eines bestimmten Zeitraums ermittelt. Der Tagesdosiszähler kann diese Anzahl oder einen diese Anzahl repräsentierenden Indikator ausgeben oder übermitteln. Alternativ oder zusätzlich kann der Tagesdosiszähler zwar die Anzahl von Benutzungen bzw. die Gesamtdosis innerhalb des Zeitraums ermitteln, zur Anzeige jedoch lediglich einen Indikator aufweisen oder ausgeben, der beispielsweise ein Erreichen oder ein Überschreiten eines Sollwerts für die Anzahl von Benutzungen bzw. die Gesamtdosis innerhalb des Zeitraums anzeigt.

Dies ist insbesondere dann hilfreich, wenn beispielsweise für eine Therapie eine Vielzahl von täglichen Benutzungen erforderlich ist, da durch den Teilzähler die Anzahl der erfolgten Benutzungen leicht nachgehalten werden kann. Gleichzeitig kann mittels des Gesamtzählers sichergestellt werden, dass in dem Behälter noch eine ausreichende Menge an zu zerstäubender Substanz vorhanden bzw. verfügbar ist, so dass eine sichere Benutzung des Zerstäubers ermöglicht ist.

Gemäß einem weiteren, auch unabhängig realisierbaren Aspekt der vorliegenden Offenbarung ist der Teilzähler zur Ermittlung einer Anzahl von innerhalb eines dem Teilzähler vorgegebenen oder vorgebbaren Zeitfensters erfolgten Benutzungen des Zerstäubers und zur Ausgabe von Informationen über die innerhalb des Zeitfensters erfolgten und/oder zu erfolgenden Benutzungen ausgebildet.

Dies ermöglicht eine Überwachung bzw. ein Monitoring der Einnahme von Medikamenten durch einen Benutzer des Zerstäubers. Zudem ermöglicht der vorschlagsgemäße Teilzähler beispielsweise eine Anzeige der täglich erfolgten Benutzungen oder der Benutzungen, die insbesondere entsprechend einem Therapieplan bzw. einer Benutzungsvorgabe noch zu erfolgen sind. Die Ausgabe kann in Form eines beispielsweise digital angezeigten oder übermittelten Zählwerts und/oder anhand eines Indikators wie einer Anzeige mit unterschiedlichen Farben, Symbolen o. dgl. erfolgen. So kann einem Benutzer bzw. Patienten die korrekte Einnahme von Medikamenten, insbesondere gemäß eines Therapieplans bzw. einer Benutzungsvorgabe, erleichtert werden und/oder die Therapietreue bzw. Compliance gefördert oder verbessert werden.

Im Folgenden sind die Begriffe "Therapieplan" und "Benutzungsvorgabe" synonym zu verstehen, sie bedeuten also im Rahmen der vorliegenden Offenbarung das gleiche. Die Begriffe "Therapieplan" und "Benutzungsvorgabe" sind insbesondere austauschbar und können jeweils anstelle des anderen Begriffs verwendet werden.

Eine Benutzungsvorgabe ist eine Vorgabe, in der festgehalten ist, wie der Zerstäuber von dem Benutzer bzw. Patienten zu benutzen ist. Insbesondere gibt eine Benutzungsvorgabe vor, zu welchen Zeitpunkten, in welchen zeitlichen Abständen, wie häufig und/oder in welcher Frequenz, insbesondere innerhalb eines bestimmten Zeitfensters wie etwa einem Tag, ein Patient bzw. Benutzer den Zerstäuber benutzen soll bzw. mittels des Zerstäubers ein Medikament inhalieren soll. Beispielsweise kann eine Benutzungsvorgabe die Vorschrift sein oder aufweisen, mehrmals täglich, insbesondere zu festgelegten Zeiten bzw. Zeitpunkten bzw. in bestimmten Zeitabständen, mit dem Zerstäuber ein Medikament einzunehmen bzw. zu inhalieren. Je nach Benutzungsvorgabe bzw. Therapieplan ist es z. B. möglich, dass der Benutzer bzw. Patient dreimal oder viermal täglich jeweils eine oder zwei Dosen inhalieren soll, also den Zerstäuber entsprechend oft benutzen bzw. betätigen und, vorzugsweise, das hierdurch erzeugte Aerosol einatmen soll. Durch Befolgen des

Therapieplans bzw. der Benutzungsvorgabe wird eine optimale Behandlung bzw. Therapie erreicht oder gewährleistet.

Insbesondere ist unter einer "zu erfolgenden Benutzung" im Sinne der vorliegenden Offenbarung eine (noch nicht erfolgte) Benutzung gemäß der Benutzungsvorgabe zu verstehen und/oder eine Benutzung, die gemäß der Benutzungsvorgabe (zukünftig) erfolgen soll bzw. vorgesehen ist.

Der Teilzähler und/oder die Detektionseinrichtung sind vorzugsweise dazu ausgebildet, eine relative Bewegung von Gehäuseteilen des Zerstäubers zueinander, insbesondere eine Drehung der Gehäuseteile gegeneinander, zu detektieren und vorzugsweise als Benutzung zu zählen. Vorzugsweise ist durch die relative Bewegung der Gehäuseteile zueinander einem (mechanischen) Energiespeicher Energie hinzufügbar bzw. der Energiespeicher ladbar, insbesondere eine Feder spannbar, vorzugsweise wobei der Energiespeicher zum Antrieb eines Druckerzeugers des Zerstäubers ausgebildet ist. Dies ermöglicht ein genaues und zuverlässiges Zählen von Benutzungen des Zerstäubers.

Ein solcher Vorgang, bei dem (bevorzugt durch Spannen des als Feder ausgebildeten Energiespeichers) dem Energiespeicher Energie hinzugefügt wird bzw. Energie in den mechanischen Energiespeicher eingebracht wird, wird auch als Ladevorgang bzw. als Laden des Energiespeichers bezeichnet. Ein Ladevorgang ist also insbesondere ein Spannvorgang, bei dem ein als Energiespeicher fungierende Feder des Zerstäubers gespannt wird. Insbesondere ist ein solcher Ladevorgang auch eine Benutzung des Zerstäubers im Sinne der vorliegenden Offenbarung.

Die Detektionseinrichtung weist vorzugsweise zwei Kopplungselemente auf, die zur vorzugsweise mechanischen Kopplung mit unterschiedlichen Gehäuseteilen des Zerstäubers ausgebildet sind. Die Kopplungselemente sind vorzugsweise derart angeordnet und/oder dazu ausgebildet, dass eine Bewegung, insbesondere Drehung, der Gehäuseteile gegeneinander mittels der Kopplungselemente auf den Teilzähler übertragbar ist. Auf diese Weise sind eine zuverlässige Kopplung der Detektionseinrichtung mit dem Zerstäuber und ein zuverlässiges Zählen sichergestellt.

Vorzugsweise ist der Teilzähler - insbesondere durch Aufschieben - mit einem Gehäuseteil bzw. einem Gehäuse des Zerstäubers verbindbar. Durch das Verbinden bzw. Aufschieben ist der Teilzähler vorzugsweise derart mit dem Zerstäuber koppelbar, dass Benutzungen des Zerstäubers mit der Detektionseinrichtung detektierbar sind. Das Koppeln bzw. Verbinden des Teilzählers mit dem Zerstäuber ist also sehr einfach und unkompliziert. Ferner kann auf diese Weise der Teilzähler dazu ausgebildet sein, mit mehreren oder verschiedenen, insbesondere gleichartigen bzw. baugleichen, Zerstäubern verwendet zu werden. Insbesondere ist der Teilzähler so austauschbar und/oder wiederverwendbar. Auf diese Weise können bei einem notwendigen Austausch des Zerstäubers Kosten gespart werden, indem der Teilzähler beibehalten und auch mit dem ausgetauschten bzw. neuen Zerstäuber weiter verwendet wird. Des Weiteren ist so ein Nachrüsten eines Zerstäubers mit einem (Teil-)Zähler möglich.

Der Teilzähler und/oder die Detektionseinrichtung sind vorzugsweise dazu ausgebildet, Auslösevorgänge und/oder Spannvorgänge als Benutzungen des Zerstäubers zu erfassen und/oder zu zählen. Dies ermöglicht eine zuverlässige Detektion und/oder Zählung von Benutzungen des Zerstäubers.

Der Teilzähler ist vorzugsweise ringartig ausgebildet und/oder dazu ausgebildet, in einer Gebrauchslage ein Gehäuseteil des Zerstäubers zumindest teilweise zu umgreifen oder zu umschließen. Vorzugsweise umschließt der Teilzähler ein Gehäuseteil des Zerstäubers in der Gebrauchslage vollständig. Dies ermöglicht eine zuverlässige Kopplung des Teilzählers mit dem Zerstäuber, eine einfache Konstruktion des Zählers, ein einfaches Anbringen an und/oder Abnehmen von dem Zerstäuber und/oder ein sicheres Halten des Zählers an dem Zerstäuber.

Vorzugsweise weist der Teilzähler ein Betätigungselement auf, wobei durch Betätigung des Betätigungselements der Teilzähler zurücksetzbar ist. Insbesondere kann so ein Patient bzw. ein Benutzer des Zerstäubers den Teilzähler selbst und/oder manuell zurücksetzen. So ist es beispielsweise möglich, den Teilzähler derart zu verwenden, dass jeweils die täglichen bzw. an einem Tag erfolgten Benutzungen gezählt werden, indem der Teilzähler jeden Tag beispielsweise am Abend und/oder nach Erreichen bzw. Erfüllen der Benutzungsvorgabe zurückgesetzt wird.

Der Teilzähler weist vorzugsweise eine - bevorzugt mechanische und/oder elektronische - Anzeigeeinrichtung zur optischen Anzeige der Informationen über die Benutzungen auf. Auf diese Weise kann ein Patient bzw. Benutzer die erfolgten und/oder noch zu erfolgenden Benutzungen von der Anzeigeeinrichtung ablesen und/oder überprüfen, ob er/sie den Zerstäuber vorschriftsmäßig benutzt, beispielsweise entsprechend einem von einem Arzt oder sonstigen medizinischen Betreuer vorgegebenen Therapieplan.

Der Teilzähler bzw. der Zerstäuber in Verbindung mit dem Teilzähler ist also vorzugsweise dazu ausgebildet, mittels der optischen Anzeige die durch den Teilzähler ermittelten Benutzungen (also die Anzahl der erfolgten Benutzungen) oder eine auf dieser Basis ermittelte Anzahl erfolgter oder noch zu erfolgender Benutzungen anzuzeigen.

Alternativ oder zusätzlich ist der Teilzähler bzw. der Zerstäuber in Verbindung mit dem Teilzähler dazu ausgebildet, mit der Anzeigeeinrichtung einen Indikator auszugeben, der Aufschluss über noch zu erfolgende oder bereits erfolgte Benutzungen erlaubt. Der Indikator kann beispielsweise eine angezeigte Anzahl, ein angezeigter Text, ein angezeigtes Symbol und/oder eine angezeigte Farbe sein. Hier sind jedoch auch andere Lösungen möglich.

Es ist also bevorzugt, jedoch nicht zwingend, dass tatsächlich auch eine Anzahl erfolgter und/oder noch zu erfolgender Benutzungen anzeigbar ist, da alternativ oder zusätzlich anzeigbar sein kann, ob bereits eine bestimmte oder ausreichende Anzahl von Benutzungen erfolgt ist und/oder ob noch Benutzungen erfolgen müssen, beispielsweise wenn die Anzahl von Benutzungen gemäß der Benutzervorgabe noch nicht erreicht ist.

Der Teilzähler kann eine Kommunikationseinrichtung zur bevorzugt drahtlosen Kommunikation aufweisen und insbesondere dazu ausgebildet sein, mit der Kommunikationseinrichtung Informationen über erfolgte und/oder zu erfolgende Benutzungen zu senden und/oder zu empfangen.

"Kommunikation" bedeutet in diesem Zusammenhang insbesondere eine elektronische Datenübermittlung, wobei die Daten entsprechende Informationen repräsentieren. Insbesondere ist der Teilzähler somit zur Kommunikation mit einem externen System zum Patientenmonitoring bzw. zur Patientenüberwachung ausgebildet sein. Das externe System kann insbesondere durch eine App oder sonstige Software gebildet sein oder diese aufweisen. Der Teilzähler kann auch einen Teil bzw. eine Hardwarekomponente eines Systems zum Patientenmonitoring bzw. zur Patientenüberwachung bilden. Dies ermöglicht eine einfache Überwachung der Benutzung des Zerstäubers und somit eine Überwachung bzw. Kontrolle, beispielsweise einer vorgesehenen Therapie. Ebenso ist es durch die oder mit der Kommunikationseinrichtung möglich, einen Therapieplan bzw. eine Benutzungsvorgabe zu ändern bzw. zu aktualisieren und/oder auf den Teilzähler zu übertragen.

Bei der Kommunikationseinrichtung kann es sich also um eine Datenschnittstelle handeln, die (unidirektional) zur Übermittlung von Zählerständen oder Zählerinformationen an externe Geräte, zum (unidirektionalen) Empfang von Steuerinformationen oder Vorgaben wie einer Benutzungsvorgabe oder eines Therapieplans, und/oder zur (bidirektionalen) Übermittlung entsprechender oder anderer Informationen ausgebildet ist.

Vorzugsweise weist der Teilzähler eine Benutzungsvorgabeeinrichtung auf. Vorzugsweise ist die Benutzungsvorgabeeinrichtung dazu ausgebildet, eine Benutzung des Zerstäubers gemäß eines Therapieplans bzw. einer Benutzungsvorgabe zu ermöglichen, zu erleichtern, zu kontrollieren und/oder sicherzustellen. Der Therapieplan bzw. die Benutzungsvorgabe kann hierbei, wie bereits weiter oben erläutert, eine vorgegebene oder vorgebbare Anzahl von Benutzungen, insbesondere pro Zeiteinheit, sein.

Die Benutzungsvorgabeeinrichtung kann als mechanische Einrichtung, als elektronische Einrichtung und/oder als Softwareeinrichtung realisiert sein.

Eine mechanische Benutzungsvorgabeeinrichtung ist vorzugsweise eine mechanische Lösung, die die Benutzungsvorgabe im Teilzähler zum Abgleich von gezählten Benutzungen mit der Benutzungsvorgabe vorgeben. Beispielsweise kann eine - insbesondere konfigurierbare - Mechanik vorgesehen sein, die bei einer vorbestimmten oder vorbestimmbaren Anzahl von Betätigungen einen Zustand bzw. eine Anzeige ändert. Dies kann durch ein Zählwerk realisiert sein, das sich nach einer bestimmten Anzahl von Betätigungen oder nach einer bestimmten oder vorbestimmten Anzahl von Betätigungen, insbesondere von innerhalb eines Zeitfensters erfolgten Betätigungen, einen Status ändert. Es kann sich bei der Benutzungsvorgabeeinrichtung also um ein Zählwerk handeln, das sich einmal pro Tag zurücksetzt oder ein Mal pro Tag zurückgesetzt wird und gemäß der Benutzungsvorgabe beispielsweise nach drei oder vier Betätigungen einen Status ändert bzw. eine Statusänderung anzeigt. Alternativ oder zusätzlich wird dies jedoch elektronisch realisiert.

Bei einer elektronischen Realisierung der Benutzungsvorgabeeinrichtung und/oder bei einer Realisierung der Benutzungsvorgabeeinrichtung mittels einer Software ist die Benutzungsvorgabe vorzugsweise auf einem Speicher des Teilzählers hinterlegt, etwa in Form einer Tabelle oder einer sonstigen geeigneten Daten- oder Software-Stru ktu r.

Insbesondere ist der Teilzähler - bei einer elektronischen Realisierung der Benutzungsvorgabeeinrichtung und/oder bei einer Realisierung der Benutzungsvorgabeeinrichtung mittels einer Software - dazu ausgebildet, die erfolgten und/oder zu erfolgenden Benutzungen mit der Benutzungsvorgabe automatisch abzugleichen. So kann auf einfache Weise direkt mittels des Zerstäubers bzw. Teilzählers das Einhalten der Benutzungsvorgabe kontrolliert und/oder ein Abweichen von der Benutzungsvorgabe bzw. dem Therapieplan festgestellt werden, insbesondere ohne dass dazu ein manuelles Aufzeichnen der Benutzungen und/oder eine Kontrolle durch medizinisches bzw. Betreuungspersonal erforderlich sind. Zudem kann ein Patient bzw. Benutzer ggf. schneller darauf aufmerksam gemacht werden, dass seine Benutzung von der Benutzungsvorgabe abweicht. Dadurch können medizinische Risiken, die durch eine von der Benutzungsvorgabe abweichende Benutzung des Zerstäubers hervorgerufen werden, vermieden oder minimiert werden.

Bevorzugt weist der Teilzähler einen Signalgeber auf, der dazu ausgebildet ist, in Abhängigkeit von dem Ergebnis des Abgleichs von erfolgten und/oder zu erfolgenden Benutzungen mit der Benutzungsvorgabe ein Signal auszugeben. Das Signal ist vorzugsweise ein optisches und/oder akustisches Signal. Es ist jedoch auch möglich, dass das Signal ein elektronisches Signal ist, insbesondere wobei das elektronische Signal an ein Mobilgerät, insbesondere ein Handy bzw. Smartphone, ein Tablet oder einen Laptop, weitergegeben wird und dort vorzugsweise akustisch und/oder optisch ausgegeben wird. Insbesondere kann ein Benutzer bzw. Patient mittels des Signals automatisch und/oder unverzüglich informiert und/oder gewarnt werden, wenn seine Benutzung des Zerstäubers von der Benutzungsvorgabe abweicht. Beispielsweise kann der Benutzer bzw. Patient so an eine geplante, notwendige und/oder vorgesehene Benutzung erinnert werden. Dies erleichtert eine sichere und vorschriftsmäßige Benutzung des Zerstäubers bzw. ein Einhalten eines verschriebenen Therapieplans. Ebenfalls kann so eine regelmäßige Einnahme der zu zerstäubenden Substanz unterstützt und/oder erleichtert werden.

Der Teilzähler ist vorzugsweise dazu ausgebildet, Zeitpunkte von und/oder zeitliche Abstände zwischen Benutzungen zu ermitteln. So ist ein sehr genauer Abgleich mit der Benutzungsvorgabe bzw. eine sehr genaue Kontrolle der Benutzung(en) des Zerstäubers möglich. Dies hilft dabei, eine optimale Therapie zu gewährleisten.

Der Teilzähler weist vorzugsweise eine Zeitbasis, insbesondere Uhr, auf. Der Teilzähler kann mittels der Zeitbasis Abstände zwischen erfolgten Benutzungen, eine seit der letzten Benutzung verstrichene Zeit und/oder die Anzahl von Benutzungen innerhalb des vorgegebenen oder vorgebbaren Zeitfensters ermitteln, kontrollieren und/oder signalisieren.

Beispielsweise kann die Notwendigkeit einer weiteren Benutzung durch ein insbesondere akustisches, optisches oder sonstiges, durch menschliche Sinne wahrnehmbares Signal ausgegeben werden. Entsprechendes kann alternativ oder zusätzlich für noch zu erfolgende oder bereits erfolgte Benutzungen innerhalb des vorgegebenen oder vorgebbaren Zeitfensters erfolgen bzw. ermöglicht werden. Alternativ oder zusätzlich kann der Teilzähler nach Ablauf einer vordefinierten Zeitspanne automatisch zurückgesetzt werden. Wenn zu diesem Zeitpunkt die vorgegebene Anzahl von Benutzungen für die vergangene Zeitspanne nicht erreicht oder überschritten ist, kann ein Fehler ausgegeben, signalisiert, übermittelt und/oder abgespeichert werden.

Gemäß einem weiteren, auch unabhängig realisierbaren Aspekt betrifft die vorliegende Offenbarung einen Zerstäuber zur Erzeugung eines einatembaren Aerosols mit einem Behälter für eine zu zerstäubende Substanz und einem Teilzähler zum Ermitteln von Benutzungen des Zerstäubers. Vorzugsweise weist der Teilzähler einen oder mehrere der vorgenannten Aspekte auf.

Ein "Behälter" im Sinne der vorliegenden Offenbarung ist vorzugsweise eine einen Innenraum bildende Aufbewahrungseinrichtung, wobei in dem Innenraum mindestens eine, bevorzugt jedoch mehrere Dosen der zu zerstäubenden Substanz aufgenommen sind oder werden können. Es handelt sich insbesondere um eine mit einer flüssigen Arzneimittelzubereitung gefüllte Kartusche, aus der sukzessive Teilmengen (Dosen) entnommen werden können, die bei der jeweiligen Benutzung des Zerstäubers in ein Aerosol überführt werden.

Der Begriff "Behälter" im Sinne der vorliegenden Offenbarung ist jedoch vorzugsweise breit zu verstehen und umfasst vorzugsweise auch andersartig gebildete Aufbewahrungseinrichtungen für oder aufweisend zu zerstäubende Substanzen. Auch hierbei weist der Behälter vorzugsweise ausreichend Substanz für mehrere Benutzungen des Zerstäubers auf. Bei dem Behälter kann es sich jedoch auch um eine Einrichtung mit mehreren einzelnen Aufnahmen für Einzeldosen handeln, wie sie im Zusammenhang mit der Zerstäubung von Pulver, insbesondere bei Pulverinhalatoren, üblich sind.

Gemein haben Behälter im Sinne der vorliegenden Offenbarung hierbei, dass sie dazu ausgebildet sind, eine ausreichende Menge (Anzahl Dosen) an Substanz für mehrere, insbesondere eine bestimmte, Anzahl von Benutzungen (Abgabe von Dosen) des Zerstäubers vorzuhalten bzw. aufzunehmen, so dass bei der jeweiligen Benutzung des Zerstäubers eine Teilmenge hiervon in Aerosol überführt wird.

Der Teilzähler weist eine Detektionseinrichtung zum Detektieren von Benutzungen des Zerstäubers auf. Vorzugsweise ist die Detektionseinrichtung eine mechanische Detektionseinrichtung, es sind aber auch elektronische und/oder elektromechanische Lösungen bzw. Detektionseinrichtungen möglich. Insbesondere ist die Detektionseinrichtung zur Detektion von Auslösevorgängen, Spannvorgängen und/oder Ladevorgängen ausgebildet, wodurch Benutzungen des Zerstäubers detektierbar sind. Vorzugsweise wird (jeweils genau) ein Auslösevorgang, Spannvorgang und/oder Ladevorgang als Benutzung detektiert und/oder gezählt. So ist ein Zählen und/oder Ermitteln von Benutzungen auf einfache und zuverlässige Weise möglich.

Gemäß einem Aspekt weist der Zerstäuber zusätzlich zu dem Teilzähler einen Gesamtzähler auf, mit dem die insgesamt mit dem Behälter erfolgten Benutzungen erfassbar sind. Vorzugsweise entspricht die Anzahl der von dem Gesamtzähler erfassten bzw. gezählten Benutzungen bzw. die Anzahl der insgesamt mit dem Behälter erfolgten Benutzungen der Anzahl von Dosen des Aerosols, die mit dem Behälter erzeugt wurden.

Vorzugsweise ist der Gesamtzähler nicht von dem Zerstäuber abnehmbar oder trennbar. Der Gesamtzähler ist also vorzugsweise fest in den Zerstäuber integriert.

Der Teilzähler und der Gesamtzähler sind vorzugsweise dazu ausgebildet, dieselben Benutzungen zu erfassen, vorzugsweise wobei die von dem Teilzähler und dem Gesamtzähler erfassten Benutzungen von derselben Detektionseinrichtung detektiert werden. Dies ermöglicht einen einfachen und kostengünstigen Aufbau des Teilzählers, wobei eine effiziente Detektion, Erfassung, Ermittlung und/oder Zählung von Benutzungen möglich ist.

Der Gesamtzähler weist vorzugsweise eine Anzeigeeinrichtung zur Anzeige von bereits erfolgten und/oder noch möglichen Benutzungen auf. Besonders bevorzugt ist der Gesamtzähler innerhalb eines zumindest abschnittsweise durchsichtigen Gehäuseteils angeordnet, so dass die Anzeigeeinrichtung (von einem Benutzer) ablesbar ist. Dies ermöglicht einem Patienten bzw. Benutzer des Zerstäubers eine einfache Kontrolle der korrekten Anwendung (z.B. gemäß der Benutzungsvorgabe) und/oder ein rechtzeitiges Wechseln des Behälters, so dass eine sichere und effektive Benutzung des Zerstäubers möglich ist.

Insbesondere ist der Gesamtzähler dazu ausgebildet, die in dem Zerstäuber bzw. dessen Behälter noch verfügbaren Dosen, also entsprechend noch möglichen Benutzungen anzuzeigen. Hierbei muss es sich nicht zwingend um eine Anzahl handeln. Beim Gesamtzähler kann es sich auch um einen Indikator handeln, der lediglich eine optische oder sonstige Anzeige über bereits erfolgte und/oder noch mögliche Benutzungen unterstützt, auch ohne dass die exakte Anzahl auf Basis der Anzeige ermittelbar ist. Vorzugsweise dient der Gesamtzähler als Gesamtdosiszähler bzw. ist der Gesamtzähler als Gesamtdosiszähler ausgebildet.

Der Teilzähler ist vorzugsweise an einer Grenze zwischen zwei Gehäuseteilen des Zerstäubers angeordnet oder anordenbar, wobei der Teilzähler mit beiden Gehäuseteilen überlappt und/oder zumindest bei einer relativen Bewegung der Gehäuseteile zueinander mit den Gehäuseteilen in Kontakt gerät bzw. gebracht wird, vorzugsweise so dass mit dem Teilzähler eine relative Bewegung der Gehäuseteile zueinander, insbesondere eine Drehung der Gehäuseteile gegeneinander, detektierbar und/oder als Benutzung des Zerstäubers zählbar ist. Dies ermöglicht ein zuverlässiges Zählen von Benutzungen des Zerstäubers.

Der Zerstäuber weist vorzugsweise einen insbesondere mechanischen Druckerzeuger auf, wobei mittels des Energiespeichers bzw. der Feder des Druckerzeugers die zu zerstäubende Substanz unter Druck setzbar und über eine Düse bzw. Austragsdüse als einatembares Aerosol ausgebbar ist. Das Aerosol kann so auf einfache, energiesparende und zuverlässige Art und Weise erzeugt werden.

Der Teilzähler ist vorzugsweise dazu ausgebildet, eine Bewegung, insbesondere ein Verdrehen, von Gehäuseteilen gegeneinander, eine axiale Position bzw. eine Veränderung der axialen Position des Behälters, eine Bewegung des Behälters, einen Zustand eines Energiespeichers bzw. Spannzustand einer Feder, eine Betätigung eines Auslösers, einen Austrag des Aerosols und/oder ein Einatmen des Aerosols als Benutzung zu erfassen. So ist ein zuverlässiges Ermitteln von Benutzungen des Zerstäubers sichergestellt.

Gemäß einem weiteren, auch unabhängig realisierbaren Aspekt betrifft die vorliegende Offenbarung ein System mit einem Zerstäuber und einer (von dem Zerstäuber separaten bzw. separierbaren) Dockingstation (bzw. Ladestation), vorzugsweise wobei die Dockingstation zum Laden eines (elektrischen) Energiespeichers des Teilzählers und/oder des Zerstäubers und/oder zur Kommunikation mit dem Teilzähler und/oder dem Zerstäuber ausgebildet ist. Der Zerstäuber und die Dockingstation weisen vorzugsweise jeweils eine Kommunikationseinrichtung zur bevorzugt drahtlosen Kommunikation auf, so dass durch die Kommunikationseinrichtungen Informationen über erfolgte und/oder zu erfolgende Benutzungen, also insbesondere insgesamt mit dem Behälter erfolgte Benutzungen, innerhalb des Zeitfensters erfolgte Benutzungen, hieraus ermittelte, noch zu erfolgende Benutzungen und/oder Benutzungsvorgaben.

So ist es beispielsweise möglich, dass die Dockingstation zur Eingabe bzw. Übermittlung der Benutzungsvorgabe ausgebildet ist. Alternativ oder zusätzlich ist die Dockingstation zum Empfang, zur Ausgabe, insbesondere Anzeige, und/oder Übermittlung eines oder mehrerer der Werte oder hierzu korrespondierender Indikatoren des Teilzählers und/oder Gesamtzählers ausgebildet.

Alternativ oder zusätzlich zum Zerstäuber kann die Dockingstation also eine oder mehrere der folgende Angaben empfangen, abrufen und/oder anzeigen: Eine, insbesondere mit dem Behälter, abgegebene oder noch abgebbare Anzahl von Dosen bzw. eine dieser entsprechenden Anzahl von bereits erfolgten und/oder noch möglichen Benutzungen des Zerstäubers und eine Anzahl von in dem vorgebbaren oder vorgegebenen Zeitfensters erfolgten oder noch zu erfolgenden Benutzungen. Eine erleichterte und/oder sichere Bedienung des Zerstäubers ist so ermöglicht.

Gemäß einem weiteren, auch unabhängig realisierbaren Aspekt betrifft die vorliegende Offenbarung ein Verfahren zum Zählen von Benutzungen eines Zerstäubers.

Gemäß einem ersten Aspekt werden bei dem vorgeschlagenen Verfahren Benutzungen des Zerstäubers sowohl von einem Teilzähler mit einer Rücksetzeinrichtung zum Zurücksetzen des Teilzählers als auch mit einem Gesamtzähler erfasst, wobei der Gesamtzähler die insgesamt mit einem Behälter des Zerstäubers erfolgten Benutzungen erfasst. Dies ermöglicht ein zuverlässiges und/oder fehlerfreies Zählen der Benutzungen und/oder ein simultanes Zählen der Benutzungen zu verschiedenen Zwecken.

Gemäß einem weiteren, auch unabhängig realisierbaren Aspekt des vorgeschlagenen Verfahrens wird dem Teilzähler ein Zeitfenster vorgegeben, wobei eine Anzahl von innerhalb des Zeitfensters erfolgten Benutzungen des Zerstäubers ermittelt wird, und wobei Informationen über die innerhalb des Zeitfensters erfolgten und/oder zu erfolgenden Benutzungen ausgegeben werden. Dies ermöglicht eine genaue Kontrolle der Benutzungen des Zerstäubers.

Weitere Aspekte, Merkmale, Eigenschaften und Vorteile der vorliegenden Offenbarung ergeben sich aus der folgenden Beschreibung bevorzugter Ausführungsformen anhand der Zeichnungen. Es zeigt:
- Fig. 1: einen schematischen Schnitt eines Zerstäubers im ungespannten Zustand;
- Fig. 2: einen schematischen, um 90° gegenüber Fig. 1 gedrehten Schnitt des Zerstäubers im gespannten Zustand;
- Fig. 3: eine Vorderansicht eines vorschlagsgemäßen Zerstäubers mit einem Teilzähler und einer Dockingstation;
- Fig. 4: eine perspektivische Ansicht eines abnehmbarem Teilzählers;
- Fig. 5: einen schematischen Schnitt durch einen Teilzähler; und
- Fig. 6: eine vereinfachte, blockschaltbildartige schematische Darstellung des Teilzählers.

In den teilweise nicht maßstabsgerechten, nur schematischen Figuren werden für gleiche oder ähnliche Teile dieselben Bezugszeichen verwendet, wobei entsprechende oder vergleichbare Eigenschaften und Vorteile erreicht werden können, auch wenn von einer wiederholten Beschreibung der Übersichtlichkeit halber abgesehen wird.

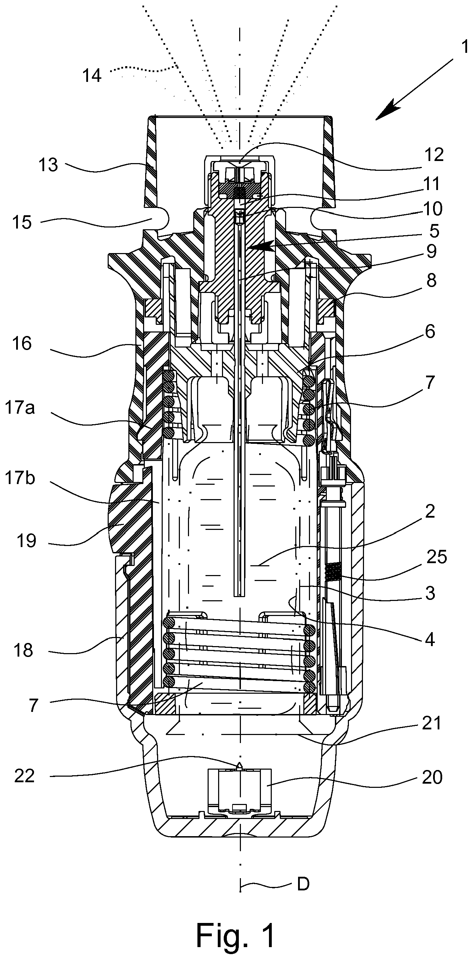

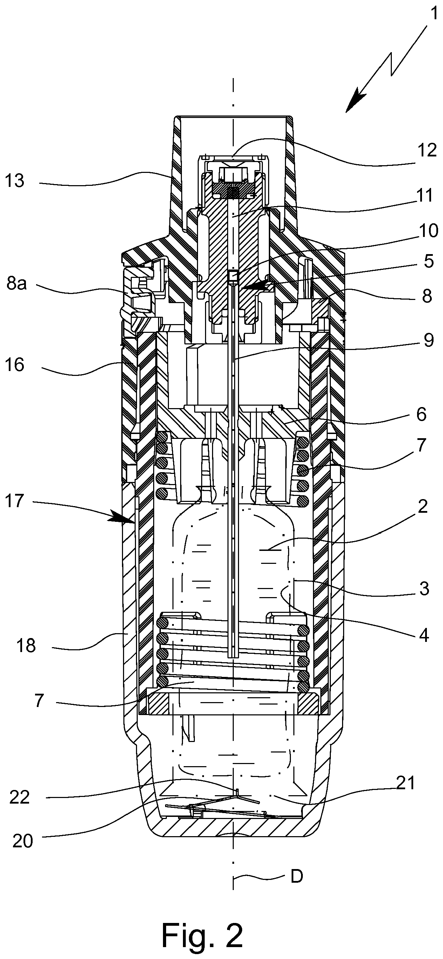

Fig. 1 und Fig. 2 zeigen einen Zerstäuber 1 zur Zerstäubung einer Substanz bzw. eines Fluids 2, insbesondere eines hochwirksamen Arzneimittels o. dgl.

Fig. 1 und Fig. 2 zeigen dabei den Zerstäuber 1 in einem schematischen Schnitt im nicht auslösebereiten oder ungespannten Zustand (Fig. 1) und im auslösebereiten oder gespannten Zustand (Fig. 2). Die Bezeichnungen "ungespannter" und "gespannter Zustand" bezeichnen hierbei den Zustand des im Zerstäuber 1 enthaltenen, vorzugsweise durch eine Feder bzw. Antriebsfeder gebildeten, Energiespeichers 7. Im "ungespannten Zustand" (Fig. 1) ist der Inhalator 1 also nicht auslösebereit und im "gespannten Zustand" (Fig. 2) ist der Zerstäuber 1 auslösebereit. Der "ungespannte Zustand" (Fig. 1) zeigt den Zerstäuber 1 im ausgelösten (nicht auslösebereiten) Zustand. Der gespannte bzw. ungespannte Zustand bzw. Zustand des Inhalators 1 entspricht also insbesondere dem (Spann-)Zustand des Energiespeichers 7 bzw. der Antriebsfeder des Inhalators 1.

Der Zerstäuber 1 ist vorzugsweise als tragbarer, portabler und/oder mobiler Zerstäuber 1 ausgebildet.

Bei Zerstäubung der Substanz bzw. des Fluids 2, vorzugsweise einer Flüssigkeit, insbesondere eines Arzneimittels, mittels des Zerstäubers 1 wird vorzugsweise ein Aerosol 14 gebildet, insbesondere wobei das Aerosol 14 von einem nicht dargestellten Benutzer eingeatmet bzw. inhaliert werden kann.

Üblicherweise erfolgt das Inhalieren wenigstens einmal täglich, insbesondere mehrmals täglich, vorzugsweise in vorbestimmten Zeitabständen, beispielsweise in Abhängigkeit von der Erkrankung des Patienten.

Bei dem Aerosol 14 handelt es sich vorzugsweise um ein Partikel-Luftgemisch mit festen und/oder flüssigen Partikeln, die besonders bevorzugt lungengängig sind, also insbesondere jedenfalls teilweise oder im Durchschnitt kleiner als 5 µm im Durchmesser sind.

Der Zerstäuber 1 ist vorzugsweise treibgasfrei, arbeitet also vorzugsweise ohne Treibgas. Stattdessen arbeitet der Zerstäuber 1 vorzugsweise mit einem mechanischen Druckerzeuger 5 bzw. Mechanismus zur Erzeugung des Aerosols 14. Insbesondere handelt es sich hierbei um eine mechanisch betriebene Pumpe, die das Fluid 2 unter Druck setzt, wodurch beim Austrag aus einer Austragsdüse 12 das Aerosol 14 erzeugt wird. Grundsätzlich kann der Zerstäuber 1 jedoch auch auf andere, bevorzugt mechanische Weise, angetrieben werden.

Der Zerstäuber 1 weist einen vorzugsweise einsetzbaren und vorzugsweise auswechselbaren Behälter 3 mit der Substanz bzw. dem Fluid 2 auf. Der Behälter 3 bildet vorzugsweise ein Reservoir für die zu zerstäubende Substanz bzw. das zu zerstäubende Fluid 2.

Vorzugsweise enthält der Behälter 3 eine ausreichende Menge an Fluid 2 bzw. Wirkstoff, um beispielsweise bis zu 200 Dosiereinheiten zur Verfügung stellen zu können, also beispielsweise bis zu 200 Zerstäubungen oder Anwendungen zu ermöglichen. Ein typischer Behälter 3, wie in der WO 96/06011 A2 oder in der WO 00/49988 A2 offenbart, nimmt ein Volumen von ca. 2 ml bis 10 ml auf.

Der Behälter 3 ist vorzugsweise zumindest im Wesentlichen zylindrisch bzw. kartuschenartig ausgebildet. Er ist im Darstellungsbeispiel von unten, nach Öffnen des Zerstäubers 1, in diesen einsetzbar und ggf. wechselbar. Er kann jedoch auch nicht auswechselbar und der Zerstäuber 1 kann ein Wegwerfprodukt sein.

Der Behälter 3 ist vorzugsweise zumindest im Wesentlichen starr ausgebildet. Besonders bevorzugt ist der Behälter 3 aus Kunststoff, insbesondere thermoplastischem Kunststoff, ganz besonders bevorzugt Polypropylen, hergestellt. Vorzugsweise weist der Behälter 3 an seinem Boden eine ebene Fläche auf oder hat der Behälter 3 einen ebenen Behälterboden 21.

Vorzugsweise ist die Substanz bzw. das Fluid 2 in einem von einem kollabierbaren Beutel gebildeten Fluidraum 4 im Behälter 3 aufgenommen.

Der Zerstäuber 1 weist vorzugsweise ferner den vorzugsweise mechanischen Druckerzeuger 5 zur Förderung und Zerstäubung der Substanz bzw. des Fluids 2, insbesondere jeweils in einer vorbestimmten, ggf. einstellbaren, Dosiermenge auf.

Der Druckerzeuger 5 weist vorzugsweise einen - im Darstellungsbeispiel nur teilweise dargestellten - Energiespeicher 7 zum Antrieb des Druckerzeugers 5 auf. Der Energiespeicher 7 ist hierbei vorzugsweise dazu ausgebildet, Energie zu speichern und an den Druckerzeuger 5 abzugeben, so dass der Druckerzeuger 5 das Fluid 2 unter Druck setzt, wodurch aus dem Fluid 2 bei Austrag aus der Austragsdüse 12 das Aerosol 14 gebildet wird. Der Druckerzeuger 5 wird also mit dem Energiespeicher 7 bzw. der durch den Energiespeicher 7 zur Verfügung gestellten Energie angetrieben.

Vorzugsweise ist der Energiespeicher 7 als Feder bzw. Schraubenfeder ausgebildet. Das Hinzufügen von Energie zu dem Energiespeicher 7 bzw. Laden des Energiespeichers 7 erfolgt insbesondere durch Zusammendrücken des Energiespeichers 7 bzw. der Feder.

Der Druckerzeuger 5 weist vorzugsweise eine Halterung 6 für den Behälter 3, den zugeordneten, nur teilweise dargestellten Energiespeicher 7, ein Förderrohr 9 mit einem Rückschlagventil 10 und/oder eine Druckkammer 11 auf.

Vorzugsweise weist der Zerstäuber ein Sperrspannwerk auf, bei dem eine Feder als Energiespeicher 7 nach Spannen von einem manuell betätigbaren Sperrelement 8 im gespannten Zustand gehalten wird, wobei die durch Spannen in der Feder gespeicherte Energie durch Betätigung des Sperrelements 8 (direkt oder vorzugsweise über eine Auslösetaste 8a) zur Druckerzeugung im Druckerzeuger 5 freigesetzt wird.

Vorzugsweise weist der Zerstäuber 1 bzw. der Druckerzeuger 5 eine Austragsdüse 12 im Bereich eines Mundstücks 13 auf. Der Behälter 3 wird vorzugsweise über die Halterung 6, insbesondere rastend, so in dem Zerstäuber 1 fixiert, dass das Förderrohr 9 in den Behälter 3 eintaucht. Die Halterung 6 kann dabei derart ausgebildet sein, dass der Behälter 3 gelöst und ausgetauscht werden kann.

Beim axialen Spannen des Energiespeichers 7 bzw. der Feder wird die Halterung 6 mit dem Behälter 3 und dem Förderrohr 9 bei den Darstellungen nach unten bewegt und das Fluid 2 aus dem Behälter 3 über das Rückschlagventil 10 in die Druckkammer 11 des Druckerzeugers 5 gesaugt.

Beim anschließenden Entspannen des Energiespeichers 7 bzw. der Feder nach oder Auslösen durch Betätigung des Sperrelements 8 wird das Fluid 2 in der Druckkammer 11 unter Druck gesetzt, indem das Förderrohr 9 mit seinem nun geschlossenen Rückschlagventil 10 durch Entspannen des Energiespeichers 7 bzw. der Feder wieder nach oben bewegt wird und nun als Druckstempel dient. Dieser Druck treibt das Fluid 2 durch die Austragsdüse 12 aus, wobei es in das Aerosol 14 zerstäubt wird, wie in Fig. 1 angedeutet.

Vorzugsweise ist das Förderrohr 9, insbesondere über die Halterung 6, in Gebrauchslage gegenüber dem Behälter 3 festgelegt. Es ist also insbesondere vorgesehen, dass eine (axiale) Bewegung des Förderrohrs 9 einer (axialen) Bewegung des Behälters 3 entspricht.

Wenn das Förderrohr 9 bzw. das Rückschlagventil 10 als Druckstempel fungiert, korrespondiert eine Bewegung des Förderrohrs 9 bzw. des Behälters 3 zu einem in der Druckkammer 11 verdrängten Volumen bzw. einer ausgetragenen oder austragbaren Fluidmenge.

Ein nicht dargestellter Benutzer bzw. Patient kann das zerstäubte Fluid 2 bzw. das Aerosol 14 inhalieren, vorzugsweise wobei Zuluft über mindestens eine Zuluftöffnung 15 in das Mundstück 13 saugbar ist.

Der Zerstäuber 1 weist vorzugsweise zueinander bewegbare, insbesondere drehbare Gehäuseteile 16, 17, 18 auf. Ferner ist der Zerstäuber 1 vorzugsweise dazu ausgebildet, durch Bewegen bzw. Verdrehen der Gehäuseteile 16, 17, 18 zueinander dem Energiespeicher 7 Energie hinzuzufügen bzw. die Feder zu spannen, insbesondere also die Feder bzw. Antriebsfeder zu komprimieren.

Der Zerstäuber 1 weist im Darstellungsbeispiel vorzugsweise ein oberes Gehäuseteil 16 und ein demgegenüber drehbares Innenteil 17 (inneres Gehäuseteil) (vgl. Fig. 2) mit einem oberen Teil 17a und einem unteren Teil 17b (vgl. Fig. 1) auf. An dem Innenteil 17 ist vorzugsweise ein insbesondere manuell betätigbares unteres Gehäuseteil (Gehäuseunterteil)18 bzw. eine Kappe vorzugsweise mittels eines Halteelementes 19 lösbar oder unlösbar befestigt, insbesondere aufgesteckt.

Zum Einsetzen und/oder Auswechseln des Behälters 3 ist das Gehäuseunterteil bzw. untere Gehäuseteil 18 vorzugsweise vom Zerstäuber 1 lösbar. Der Behälter 3 kann jedoch auch unauswechselbarsein.

Das untere Gehäuseteil 18 kann gegen das obere Gehäuseteil 16 gedreht bzw. relativ zu dem oberen Gehäuseteil 16 verdreht werden, vorzugsweise wobei es den in der Darstellung unteren Teil 17b des Innenteils 17 mitnimmt.

Insbesondere ist das untere Gehäuseteil 18 drehfest an dem Innenteil 17 angeordnet, vorzugsweise mittels einer formschlüssigen Verbindung. Das Innenteil 17 kann auf diese Weise mittels des unteren Gehäuseteils 18 gegen das Gehäuseteil 16 gedreht bzw. relativ zu dem Gehäuseteil 16 verdreht werden. Hier sind jedoch auch andere Lösungen möglich.

Das Drehen der Gehäuseteile 16 und 17 bzw. 16 und 18 gegeneinander bzw. das relative Verdrehen der Gehäuseteile 16 und 17 bzw. 16 und 18 zueinander erfolgt um eine Drehachse D des Zerstäubers 1. Die Drehachse D bildet vorzugsweise eine Längsachse, Mittelachse und/oder Symmetrieachse des Zerstäubers 1.

Nachfolgend wird unter einer "axialen" Bewegung bzw. Richtung eine Bewegung bzw. Richtung verstanden, die identisch mit der Drehachse D ist und/oder parallel zu oder entlang dieser verläuft. Auch der Begriff "radial" bezieht sich entsprechend jeweils auf die Drehachse D.

Durch das Drehen des oberen Gehäuseteils 16 relativ zu dem unteren Gehäuseteil 18 bzw. dem Innenteil 17 wird der Energiespeicher 7 bzw. die Feder über ein nicht dargestelltes, auf die Halterung 6 wirkendes Getriebe in axialer Richtung gespannt, insbesondere zusammengedrückt. Mit dem Spannen wird der Behälter 3 vorzugsweise axial nach unten bewegt, bis der Behälter 3 eine in Fig. 2 angedeutete Endlage annimmt. In diesem Zustand ist der Energiespeicher 7 bzw. die Feder gespannt bzw. befindet sich der Zerstäuber 1 im gespannten Zustand. Während des Zerstäubungsvorgangs wird der Behälter 3 vorzugsweise von dem Energiespeicher 7 bzw. der Feder wieder (nach oben) in seine Ausgangslage zurückbewegt.

Der Behälter 3 führt vorzugsweise eine axiale Bewegung oder Hubbewegung während dem Hinzufügen von Energie bzw. während des Spannvorgangs bzw. zur Fluidentnahme und/oder während der Zerstäubung bzw. Abgabe des Fluids 2 aus.

Insbesondere steht die axiale Position des Behälters 3 also in direktem Zusammenhang mit dem Zustand bzw. Spannzustand des Inhalators 1 bzw. Energiespeichers 7 bzw. ist die axiale Position des Behälters 3 mit dem Zustand bzw. Spannzustand korreliert. Im in Fig. 1 dargestellten ungespannten Zustand befindet sich der Behälter 3 vorzugsweise in der aus Fig. 1 ersichtlichen oberen axialen Position. Im gespannten Zustand befindet sich der Behälter 3 vorzugsweise in der aus Fig. 2 ersichtlichen unteren axialen Position.

In der "oberen axiale Position" des Behälters 3 befindet sich der Behälterboden 21 näher an der Halterung 6, dem Mundstück 13, dem oberen Gehäuseteil 16 und/oder dem inneren Gehäuseteil 18 als in der "unteren axialen Position".

Vorzugsweise kommt beim erstmaligen Spannen eine axial wirkende, im Gehäuseteil 18 angeordnete Feder 20 am Behälterboden 21 zur Anlage und sticht mit einem Anstechelement 22 den Behälter 3 bzw. eine bodenseitige Versiegelung bei der erstmaligen Anlage zur Belüftung an.

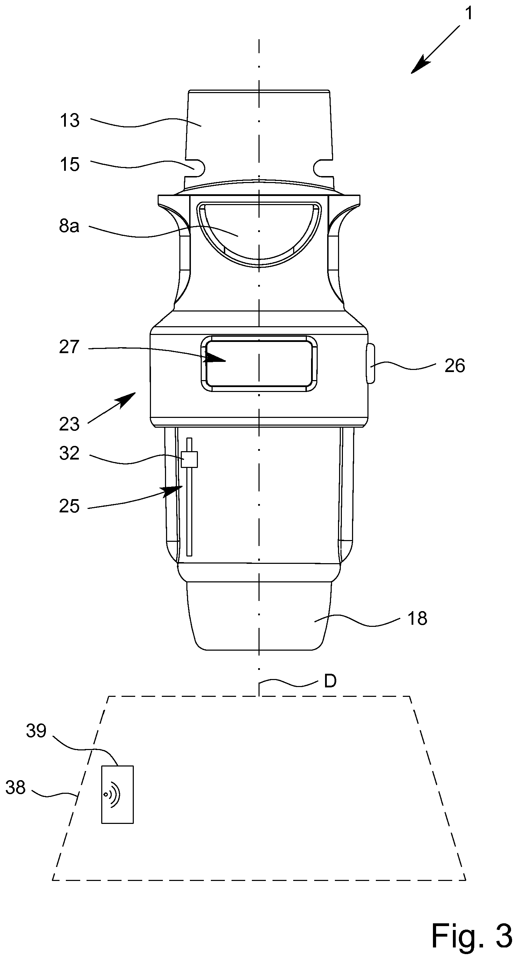

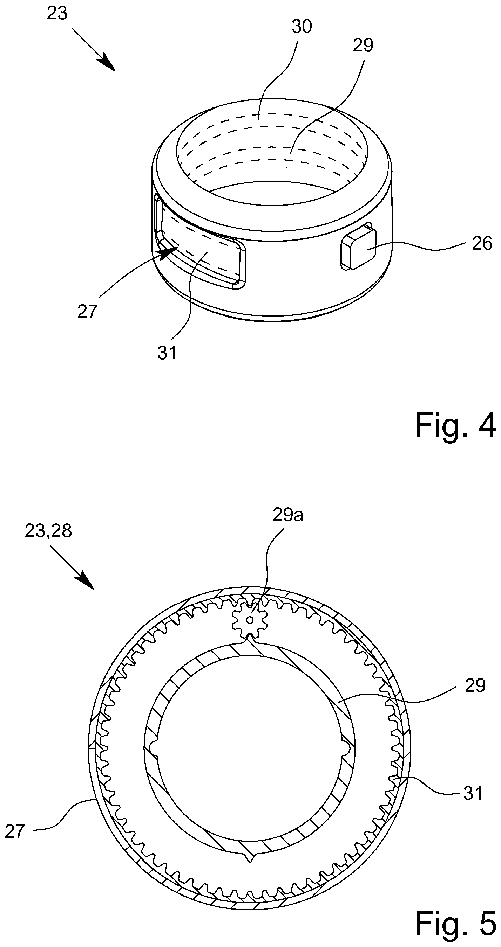

Die Fig. 3 zeigt einen vorschlagsgemäßen Zerstäuber 1 in einer Vorderansicht. Die Fig. 4 zeigt einen vorschlagsgemäßen Teilzähler 23 in einer schematischen perspektivischen Darstellung.

Der Teilzähler 23 ist grundsätzlich unabhängig von dem Zerstäuber 1 bzw. als von diesem separate Einheit realisierbar. Dies ermöglicht die Wiederverwendung des Teilzählers 23 bei Wechsel des Zerstäubers 1.

Der vorschlagsgemäße Zerstäuber 1 weist den Teilzähler 23 auf, wobei der Teilzähler 23 zur Ermittlung von Benutzungen des Zerstäubers 1 ausgebildet ist.

Vorzugsweise weist der Teilzähler 23 eine Rücksetzeinrichtung 24 zum Zurücksetzen des Teilzählers 23 auf. Die Rücksetzeinrichtung 24 ist vorzugsweise dazu ausgebildet, eine angezeigten und/oder gespeicherten Zählerwert oder Zählerstand zu verändern, insbesondere zu löschen und/oder auf "0" zu setzen bzw. zu verändern.

Unter einem "Zurücksetzen des Teilzählers 23" wird also insbesondere das Zurücksetzen, Verändern und/oder Löschen eines von dem Teilzähler 23 angezeigten und/oder gespeicherten Zählerwerts oder Zählerstands verstanden.

Das Zurücksetzen des Teilzählers 23 kann mechanisch, elektronisch und/oder elektromechanisch erfolgen, beispielsweise durch Löschen oder Zurücksetzen eines elektronisch gespeicherten Zählerwerts bzw. Zählerstands oder durch mechanisches Zurückstellen einer mechanischen Anzeige bzw. Anzeigeeinrichtung. Dies wird später noch genauer erläutert.

Unter einem "Zählerwert" und/oder "Zählerstand" ist insbesondere ein Wert bzw. eine Zahl zu verstehen, die der (vom Teilzähler 23 erfassten) Anzahl der Benutzungen des Zerstäubers 1, insbesondere nach einem Zurücksetzen des Teilzählers 23, entspricht. Auch wenn mit dem Teilzähler 23 also alle Benutzungen des Zerstäubers 1 erfassbar sind bzw. erfasst werden, so entspricht der Zählerwert oder Zählerstand vorzugsweise jedoch nur der Anzahl von Benutzungen nach dem letzten Zurücksetzen des Teilzählers 23. Insbesondere sind die Begriffe "Zählerwert" und "Zählerstand" im Sinne der vorliegenden Offenbarung synonym zu verstehen.

Der Zerstäuber 1 weist zusätzlich zu dem Teilzähler 23 vorzugsweise einen Gesamtzähler 25 auf, wobei mit dem Gesamtzähler 25 die insgesamt mit dem Behälter 3 erfolgten Benutzungen erfassbar sind.

Vorzugsweise ist der Gesamtzähler 25 nicht oder nur durch Austausch des Behälters 3 zurücksetzbar. Der Gesamtzähler 25 ist vorzugsweise dazu ausgebildet, die bereits insgesamt mit dem Behälter 3 erfolgten Benutzungen und/oder die mit dem Behälter 3 noch möglichen Benutzungen anzuzeigen. Insbesondere ist die Anzahl der noch möglichen Benutzungen durch die Menge des noch in dem Behälter 3 vorhandenen Fluids 2 bestimmt oder bedingt.

Der Teilzähler 23 weist vorzugsweise ein Betätigungselement 26 auf. Das Betätigungselement 26 ist dazu ausgebildet und/oder derart angeordnet, dass durch, insbesondere manuelle, Betätigung des Betätigungselements 26 der Teilzähler 23 zurücksetzbar ist. Vorzugsweise weist die Rücksetzeinrichtung 24 das Betätigungselement 26 auf.

Das Betätigungselement 26 kann als Taste, Knopf, Schalter und/oder Betätigungsfeld ausgebildet sein, die/der/das manuell von einem nicht dargestellten Benutzer betätigbar, insbesondere drückbar, ist. Hierdurch ist ein Zurücksetzen des Teilzählers 23 auslösbar.

Vorzugsweise ist das Betätigungselement 26 mit der Rücksetzeinrichtung 24 gekoppelt und/oder verbunden, bevorzugt sodass durch eine Betätigung des Betätigungselements 26 die Rücksetzeinrichtung 24 gesteuert oder bedient wird bzw. steuerbar oder bedienbar ist. Besonders bevorzugt bewirkt ein Betätigen des Betätigungselements 26 ein Zurücksetzen des Teilzählers 23 mittels der Rücksetzeinrichtung 24.

Eine Betätigung des Betätigungselements 26 und/oder ein Zurücksetzen der oder durch die Rücksetzeinrichtung 24 ist oder erfolgt vorzugsweise unabhängig von einem Öffnen oder Schließen des Zerstäubers 1, einem Wechseln des Behälters 3, und/oder einem Abnehmen des (unteren) Gehäuseteils 18 bzw. einer Kappe des Zerstäubers 1. Insbesondere ist ein Betätigen des Betätigungselements 26 und/oder ein Zurücksetzen der Rücksetzeinrichtung 24 unabhängig von einem Zustand oder Betriebszustand des Zerstäubers 1 und/oder eines Zustands bzw. einer Füllmenge des Behälters 3 und/oder zu jedem beliebigen Zeitpunkt möglich.

Der Teilzähler 23 weist vorzugsweise eine Anzeigeeinrichtung 27 auf, die zur optischen Anzeige von Informationen über Benutzungen des Zerstäubers 1 ausgebildet ist. Vorzugsweise ist die Anzeigeeinrichtung 27 elektronisch betrieben und/oder betreibbar. Die Anzeigeeinrichtung 27 kann beispielsweise eine LED sein, aber auch als Bildschirm oder Display ausgebildet sein. Bevorzugt ist die Anzeigeeinrichtung 27 als LED- oder LCD-Display ausgebildet, vorzugsweise wobei die Anzeigeeinrichtung 27 zur farbigen Anzeige der Informationen ausgebildet ist. Alternativ oder zusätzlich handelt es sich bei der Anzeigeeinrichtung 27 um ein mechanisch antreibbares Anzeigeelement wie eine Skala, ein Zählrad o. dgl.

Die Anzeigeeinrichtung 27 ist bevorzugt zur Anzeige des Zählerstands ausgebildet. Dabei sind verschiedene Varianten der Anzeige des Zählerstands möglich. Beispielsweise ist es möglich, dass eine Zahl angezeigt wird, die dem Zählerstand entspricht. Die Informationen über die Benutzungen des Zerstäubers 1 können jedoch auch auf andere Weise ausgebbar bzw. mittels der Anzeigeeinrichtung 27 darstellbar sein, etwa indem mittels der Anzeigeeinrichtung 27 ein sich mit der Anzahl der Benutzungen bzw. dem Zählerstand ändernder Indikator, insbesondere ein sich in der Länge verändernder Balken angezeigt wird. Ebenso ist es möglich, dass die Informationen in einer bestimmten Farbe angezeigt werden, z. B. grün und/oder rot, insbesondere um einen Benutzer so entsprechende Hinweise zu geben. Die Anzeigeeinrichtung 27 kann auch dazu ausgebildet sein, Texte anzuzeigen.

Vorzugsweise ist der Teilzähler 23 mit dem Zerstäuber 1 bzw. dessen Gehäuse bzw. einem der Gehäuseteile 16, 17, 18 (mechanisch) koppelbar bzw. verbindbar und/oder davon trennbar bzw. abnehmbar. Der Teilzähler 23 kann also insbesondere als "add-on-device" (d.h. als nachträglich montierbares Zusatzgerät) und/oder als Nachrüstteil bzw. von dem Zerstäuber 1 separates Zubehörteil ausgebildet sein.

In einer bevorzugten Ausführungsform ist der Teilzähler 23 auf den Zerstäuber 1 bzw. dessen Gehäuse aufschiebbar bzw. durch Aufschieben und/oder eine zumindest im Wesentlichen axiale und/oder geradlinige Bewegung entlang der Drehachse D mit dem Zerstäuber 1 bzw. dessen Gehäuse verbindbar und/oder koppelbar. Vorzugsweise ist hierdurch der Teilzähler 23 derart mit dem Zerstäuber 1 koppelbar, dass Benutzungen des Zerstäubers 1 mit einer Detektionseinrichtung 28 des Teilzählers 23 detektierbar sind.

Bei der in Fig. 4 gezeigten Ausführungsform umgibt oder umschließt der Teilzähler 23 das Gehäuse des Zerstäubers 1 in Umfangsrichtung vorzugsweise vollständig, wenn der Teilzähler 23 mit dem Zerstäuber 1 verbunden, insbesondere auf den Zerstäuber 1 aufgeschoben, ist.

Der Teilzähler 23 ist vorzugsweise ringartig ausgebildet und/oder dazu ausgebildet, in einer (insbesondere in Fig. 3 dargestellten) Gebrauchslage ein Gehäuseteil 16, 17, 18 des Zerstäubers 1 zumindest teilweise, vorzugsweise vollständig, zu umgreifen oder zu umschließen. Vorzugsweise ist so ein reibschlüssiges und/oder formschlüssiges Halten des Teilzählers 23 an dem Zerstäuber 1 bzw. dessen Gehäuse ermöglicht.

Eine Gebrauchslage des Teilzählers 23 ist insbesondere eine Lage des Teilzählers 23 relativ zu dem Zerstäuber 1, in der eine Ermittlung von Benutzungen des Zerstäubers 1 mit dem bzw. durch den Teilzähler 23 ermöglicht ist. Vorzugsweise ist der Teilzähler 23 in der Gebrauchslage mit dem Zerstäuber 1 gekoppelt oder verbunden bzw. an dem Zerstäuber 1 angeordnet, insbesondere auf diesen aufgeschoben.

Vorzugsweise ist der Teilzähler 23 in der Gebrauchslage derart an dem Zerstäuber 1 angeordnet, dass Benutzungen des Zerstäubers 1 detektierbar bzw. ermittelbar sind. Insbesondere ist der Teilzähler 23 dazu in der Gebrauchslage oder zumindest bei einer Bewegung der Gehäuseteile 16, 17, 18 zueinander mit diesen gekoppelt. Vorzugsweise ist bzw. gerät der Teilzähler 23 in der Gebrauchslage, zumindest bei einer Bewegung der Gehäuseteile 16, 17, 18 zueinander, mit diesen in Kontakt und/oder überlappt der Teilzähler 23 in der Gebrauchslage mit den Gehäuseteilen 16, 17, 18.

Es ist jedoch auch möglich, dass der Teilzähler 23 in einem vorzugsweise abnehmbaren Gehäuseteil des Zerstäubers 1, vorzugsweise dem unteren Gehäuseteil 18, integriert oder fest damit verbunden oder hierdurch gebildet ist. Vorzugsweise ist in diesem Fall der Teilzähler 23 innenseitig bzw. auf der Innenseite des Gehäuseteils 18 angeordnet und/oder ist das Gehäuseteil 18 durchsichtig ausgebildet und/oder weist ein Fenster auf, so dass der Teilzähler 23 bzw. die Anzeigeeinrichtung 27 durch das Gehäuseteil 18 hindurch von einem nicht dargestellten Benutzer ablesbar ist. Es ist aber auch eine Integration des Teilzählers 23 in das Gehäuseteil 16 möglich.

Auch bei der Integration in das Gehäuseteil 16, 18 ist ein leichtes bzw. schnelles Austauschen des Teilzählers 23 und/oder ein Nachrüsten des Zerstäubers 1 mit dem Teilzähler 23 auf einfache und/oder kostengünstige Weise ermöglicht.

Vorzugsweise weist der Teilzähler 23 eine Detektionseinrichtung 28 auf, die zum Detektieren von Benutzungen des Zerstäubers 1 ausgebildet ist. Insbesondere kann das Detektieren mechanisch, elektronisch und/oder elektromechanisch erfolgen. Eine Detektion einer Benutzung des Zerstäubers 1 erfolgt besonders bevorzugt durch eine Detektion eines Auslösens bzw. Auslösevorgangs und/oder eines Spannens bzw. Spannvorgangs. Die Detektionseinrichtung 28 ist insbesondere dazu ausgebildet, eine relative Bewegung, insbesondere Drehung, der Gehäuseteile 16, 17, 18 zueinander zu detektieren. Vorzugsweise ist der Teilzähler 23 dazu ausgebildet, eine derartige detektiere Bewegung als Benutzung des Zerstäubers 1 zu zählen.

Wie bereits weiter oben erläutert, erfolgt eine Benutzung des Zerstäubers 1 vorzugsweise wie folgt bzw. umfasst die folgenden Schritte: Zunächst wird (nach einer vorherigen Benutzung) der Zerstäuber 1 in den auslösebereiten Zustand versetzt bzw. der Energiespeicher 7 bzw. die Feder gespannt. Das Spannen des Energiespeichers 7 bzw. der Feder erfolgt vorzugsweise durch eine Relativbewegung von Gehäuseteilen 16, 17, 18 insbesondere durch Drehen des Innenteils 17, besonders bevorzugt um 180°.

Vorzugsweise wird das Innenteil 17 von einem Benutzer nicht direkt betätigt bzw. gedreht, sondern mittelbar durch manuelles Drehen des unteren Gehäuseteils 18 gedreht, wobei das untere Gehäuseteil 18 den Innenteil 17 mitnimmt.

Wenn das der Energiespeicher 7 bzw. die Feder gespannt ist, befindet sich der Zerstäuber 1 im auslösebereiten Zustand. Eine Zerstäubung des Fluids 2 bzw. Auslösung erfolgt anschließend durch eine Entsperrung des Sperrelements 8. Insbesondere erfolgt die Entsperrung des Sperrelements bzw. das Auslösen durch ein (manuelles) Betätigen der Auslösetaste 8a durch den (nicht dargestellten) Benutzer.

Das Betätigen der Auslösetaste 8a bzw. des Entsperren des Sperrelements 8 bewirkt dann die Zerstäubung des Fluids 2 bzw. die Erzeugung des einatembaren Aerosols 14. Vorzugsweise wird das erzeugte Aerosol 14 von dem nicht dargestellten Benutzer eingeatmet bzw. inhaliert.

Das Spannen des Energiespeichers 7 bzw. der Feder und/oder das Auslösen mittels Entsperren des Sperrelements 8 ist vorzugsweise mittels des Teilzählers 23 bzw. der Detektionseinrichtung 28 (als Benutzung) detektierbar. Eine Detektion des Spannens des Energiespeichers 7 bzw. der Feder kann insbesondere über eine Detektion einer Drehung des Innenteils 17 und/oder des unteren Gehäuseteils 18 relativ zu dem oberen Gehäuseteil 16 erfolgen.

In Fig. 5 ist beispielhaft eine mögliche Ausführungsform einer mechanischen Detektionseinrichtung 28 dargestellt.

Vorzugsweise weist die Detektionseinrichtung 28 ein Anzeigeelement 31 zur Anzeige eines Zählerwerts oder Zählerstands oder eines hierzu korrespondierenden Indikators auf. Die zuvor bereits beschriebene Anzeigeeinrichtung 27 kann das Anzeigeelement 31 aufweisen oder bilden. Die Anzeigeeinrichtung 27 kann also zur mechanischen Anzeige ausgebildet sein. Hierbei kann die Anzeigeeinrichtung 27 ein Fenster aufweisen, durch das das Anzeigeelement 31 sichtbar angeordnet ist. Die Anzeigeeinrichtung 27 kann ferner eine Markierung aufweisen, die auf eine bestimmte Position des Anzeigeelements 31 hinweist. Hier sind jedoch auch andere Lösungen möglich.

Vorzugsweise arbeitet oder funktioniert die Detektionseinrichtung 28 in dem in Fig. 5 dargestellten Beispiel (rein) mechanisch und/oder weist die Detektionseinrichtung 28 einen (mechanischen) Mechanismus zur Detektion von Benutzungen des Zerstäubers 1 auf. Insbesondere ist mit der Detektionseinrichtung 28 die Drehung eines Gehäuseteils 16, 17, 18 bzw. das Verdrehen von Gehäuseteilen 16, 17, 18 zueinander detektierbar.

Die Detektionseinrichtung 28 weist vorzugsweise eine Kupplung auf, durch die der Teilzähler 23 mittels der Relativbewegung der Gehäuseteile 16, 17, 18 des Zerstäubers 1 antreibbar ist.

Im Darstellungsbeispiel weist die Kupplung ein erstes Kopplungselement 29 und, vorzugsweise, ein zweites Kopplungselement 30 auf. In Fig. 4 sind die Kopplungselemente 29, 30 schematisch dargestellt. Die Kopplungselemente 29, 30 sind vorzugsweise dazu ausgebildet, eine Relativbewegung, insbesondere Drehbewegung, von Gehäuseteilen 16, 17, 18 des Zerstäubers 1 derart auf den Teilzähler 23 zu übertragen, dass dieser antreibbar bzw. triggerbar ist.

Das zweite Kopplungselement 30 ist vorzugsweise zur insbesondere drehfesten Kopplung mit dem (oberen) Gehäuseteil 16 ausgebildet bzw. drehfest an dem (oberen) Gehäuseteil 16 angeordnet oder anordenbar.

Vorzugsweise sind das erste und zweite Kopplungselement 29, 30 relativ zueinander bewegbar, insbesondere gegeneinander (um eine gemeinsame Achse) drehbar.

Die Kopplungselemente 29, 30 sind vorzugsweise jeweils ringartig ausgebildet und/oder korrespondieren in ihrer Form der äußeren Form des jeweils zur Kopplung zugeordneten Gehäuseteils 16, 17, 18 des Zerstäubers 1.

Vorzugsweise weist das erste Kopplungselement 29 als Mitnehmer ausgebildet, der bei in Gebrauchslage befindlichem Teilzähler 23 durch Kopplung mit dem Gehäuseteil 16, 17, 18 des Zerstäubers 1 in entsprechender Weise bewegt, insbesondere gedreht, wird. Hier sind jedoch auch andere Lösungen möglich.

Vorzugsweise ist das erste Kopplungselement 29, insbesondere form- und/oder reibschlüssig, mit dem Innenteil 17 und/oder dem unteren Gehäuseteil 18 verbunden oder verbindbar. Das erste Kopplungselement 29 ist vorzugsweise drehfest an dem Innenteil 17 und/oder dem unteren Gehäuseteil 18 angeordnet oder anordenbar.

Vorzugsweise ist das erste Kopplungselement 29 dazu ausgebildet, eine Drehung des Innenteils 17 und/oder des unteren Gehäuseteils 18 auf das Anzeigeelement 31 des Teilzählers 23 zu übertragen bzw. das Anzeigeelement 31 durch eine Drehung des Innenteils 17 und/oder des unteren Gehäuseteils 18 anzutreiben.

Das erste Kopplungselement 29 und/oder das Anzeigeelement 31 sind vorzugsweise ringartig oder als Ring ausgebildet und/oder umgreifen das erste Kopplungselement 29 das Innenteil 17 und/oder das untere Gehäuseteil 18. Hier sind jedoch auch andere Lösungen möglich.

Der Teilzähler 23 bzw. Mechanismus weist vorzugsweise ein Getriebe zur Übertragung einer Bewegung oder Drehung eines der Gehäuseteile 16, 17, 18, insbesondere des Innenteils 17 und/oder des unteren Gehäuseteils 18, auf das Anzeigeelement 31 auf.

Vorzugsweise kämmt das erste Kopplungselement 29 bei einer Drehung des Innenteils 17 und/oder des unteren Gehäuseteils 18 mit einem Zahnrad 29a des Getriebes, wodurch sich das Zahnrad 29a des Getriebes dreht und diese Drehung wiederum das Anzeigeelement 31 antreibt. Insbesondere wird die Drehung des Innenteils 17 und/oder des unteren Gehäuseteils 18 derart übertragen, dass sich das Anzeigeelement 31 pro einer oder mehreren Benutzungen bzw. Spannvorgängen des Zerstäubers 1 um eine Einheit, insbesondere um eine Skaleneinteilung und/oder um einen bestimmten Winkel oder eine bestimmte Strecke bewegt bzw. dreht, insbesondere wobei der (angezeigte) Zählerwert sich um Eins erhöht, die Farbe wechselt o. dgl.

Das erste Kopplungselement 29 weist vorzugsweise einen Mitnehmer auf oder bildet einen Mitnehmer, der mit dem Getriebe gekoppelt oder koppelbar ist bzw. in ein Zahnrad 29a des Getriebes eingreift.

Vorzugsweise ist die Detektionseinrichtung 28 dazu ausgebildet, dass eine Drehung des Innenteils 17 und/oder des unteren Gehäuseteils 18 eine Änderung des angezeigten Inhalts, Werts, der angezeigten Position und/oder des Zählerstands oder Zählerwerts oder Indikators bzw. eine Änderung des von dem Anzeigeelement 31 angezeigten Inhalts, Werts, der angezeigten Position und/oder des Zählerwerts oder Zählerstands oder Indikators des Anzeigeelements 31 bewirkt. Der angezeigte Zählerwert kann auch derart schrittweise verändert werden, dass eine Veränderung der Anzeige nur nach einer mehrfachen Benutzung des Zerstäubers 1 erfolgt. Alternativ oder zusätzlich kann ein durch das Anzeigeelement 31 gebildeter Indikator, insbesondere inkrementell bzw. schrittweise geändert werden.

Das Anzeigeelement 31 ist vorzugsweise bewegbar, insbesondere drehbar. Hierbei ist eine Drehung des Innenteils 17 und/oder des unteren Gehäuseteils 18 mittels der Kupplung bzw. des Getriebes in eine Drehung des Anzeigeelements 31 umsetzbar, vorzugsweise mit einer Untersetzung.

Insbesondere ist das Getriebe dazu ausgebildet, eine Drehung des Innenteils 17 und/oder Gehäuseteils 18, vorzugsweise jeweils um 180°, derart auf das Anzeigeelement 31 zu übertragen, dass der von dem Anzeigeelement 31 angezeigte Zählerwert oder Indikator inkrementell bzw. schrittweise verändert, insbesondere erhöht, wird.

Das Anzeigeelement 31 ist vorzugsweise ringartig oder als Ring ausgebildet. Hierbei können (in einer Gebrauchslage des Teilzählers 23) das Innenteil 17 und/oder das untere Gehäuseteil 18 innerhalb des Rings angeordnet sein.

Vorzugsweise sind die Kopplungselemente 29, 30 und/oder das Anzeigeelement 31 koaxial zur Drehachse D angeordnet.

Vorzugsweise weist das Anzeigeelement 31 an seiner radialen Außenseite Zählerwerte bzw. den Zählerwert und/oder Indikator auf, insbesondere in Form von an dem Anzeigeelement 31 angebrachten oder auf das Anzeigeelement 31 aufgetragenen Zahlen, Farben o. dgl. Alternativ oder zusätzlich weist das Anzeigeelement 31 einen Indikator wie einen Farbverlauf, unterschiedlich farbige Abschnitte o. dgl. auf.

Zur Kopplung des Anzeigeelements 31 mit dem Getriebe weist das Anzeigeelement 31 vorzugsweise Zähne auf und/oder ist das Anzeigeelement 31 vorzugsweise zahnradartig ausgebildet, so dass mittels des Getriebes das Anzeigeelement 31 antreibbar und/oder drehbar ist. Die Zähne zur Kopplung mit dem Getriebe sind vorzugsweise innenseitig an dem Anzeigeelement 31 angeordnet.

Vorzugsweise ist das Anzeigeelement 31 derart in dem Teilzähler 23 angeordnet, dass jeweils nur eine der Zahlen für einen Benutzer des Zerstäubers 1 sichtbar, insbesondere lesbar, ist bzw. angezeigt wird. Vorzugsweise entspricht die sichtbare bzw. angezeigte Zahl dem Zählerwert.

Alternativ oder zusätzlich ist die Anzeigeeinrichtung 27 dazu ausgebildet, einen bestimmten Zählerwert oder Indikatorabschnitt zu markieren. Dies kann, wie üblich, mit einer Markierung erfolgen, die an oder neben dem Anzeigeelement 31 derart vorgesehen ist, dass auf einen Wert oder Abschnitt hingedeutet wird, insbesondere durch einen Pfeil, Strich o. dgl.

Vorzugsweise ist das Getriebe als Zahnradgetriebe ausgebildet. Das Getriebe weist vorzugsweise ein oder mehrere Zahnräder 29a und/oder eine oder mehrere Wellen zur Übertragung von Bewegungen, Drehungen und/oder Drehmomenten auf.

Vorzugsweise ist das Getriebe dazu ausgebildet, durch eine Drehung des Innenteils 17 und/oder des unteren Gehäuseteils 18 angetrieben zu werden, insbesondere beim Spannen des Energiespeichers 7 bzw. der Feder des Zerstäubers 1.

Das Getriebe kann mit dem Innenteil 17 und/oder dem unteren Gehäuseteil 18 direkt gekoppelt sein oder indirekt über das Kopplungselement 29 des Teilzählers 23 gekoppelt sein.

Die Drehrichtung, bei der sich der von dem Anzeigeelement 31 angezeigte Zählerwert erhöht, wird im Folgenden als Zählrichtung bezeichnet.

Vorzugsweise weist der Teilzähler 23 eine (nicht dargestellte) Sperrklinke auf, die dazu ausgebildet ist, eine Drehung des Anzeigeelements 31 entgegen der Zählrichtung zu verhindern. Insbesondere greift die Sperrklinke derart in das Anzeigeelement 31 ein, dass eine Drehung entgegen der Zählrichtung blockiert ist.

Vorzugsweise ist die Rücksetzeinrichtung 24 mit dem Mechanismus mechanisch gekoppelt und/oder der Mechanismus weist die Rücksetzeinrichtung 24 auf. Hierbei sind das Betätigungselement 26 und/oder die Rücksetzeinrichtung 24 dazu ausgebildet, dass durch eine Betätigung des Betätigungselements 26 das Anzeigeelement 31, insbesondere entgegen der Zählrichtung, in eine Anfangsposition oder Ausgangsposition zurückbewegt wird bzw. die Rücksetzeinrichtung 24 betätigt wird bzw. der Teilzähler 23 zurückgesetzt wird, insbesondere durch eine Rückstelleinrichtung des Teilzählers 23.

Vorzugsweise weist die Rücksetzeinrichtung 24 die bereits genannte Sperrklinke und/oder eine Feder, insbesondere eine Spiralfeder auf, wobei die Sperrklinke und/oder die Feder mechanisch mit dem Betätigungselement 26 gekoppelt sind, so dass durch Betätigung des Betätigungselements 26 ein Zurücksetzen des Anzeigeelements bzw. des Teilzählers 23 bewirkbar ist.

Vorzugsweise ist das Anzeigeelement 31 mit der Feder federbelastet, insbesondere wobei die Feder beim Drehen des Anzeigeelements 31 in Zählrichtung bzw. Erhöhen des Zählerwerts gespannt wird.

Die Sperrklinke und das Betätigungselement 26 sind vorzugsweise derart gekoppelt, dass durch eine Betätigung des Betätigungselements 26 die Blockierung des Anzeigeelements 31 entgegen der Zählrichtung durch die Sperrklinke lösbar oder aufhebbar ist.

Vorzugsweise wird das Anzeigeelement 31 mittels der Feder entgegen der Zählrichtung gedreht bzw. wird der Teilzähler 23 zurückgesetzt, wenn die Blockade durch die Sperrklinke aufgehoben ist.

Die Detektionseinrichtung 28 ist vorzugsweise an dem (oberen) Gehäuseteil 16 und/oder dem (unteren) Gehäuseteil 18 des Zerstäubers 1 angeordnet.

Eine Detektion des Drehens des Gehäuseteils 17, 18 gegen das Gehäuseteil 16 und somit eine Detektion einer Benutzung des Zerstäubers 1 kann alternativ oder zusätzlich zu der mechanischen Detektion elektrisch oder elektromechanisch erfolgen. Vorzugsweise weist die Detektionseinrichtung 28 dazu einen Mikroschalter, ein Potentiometer, einen Schleifkontakt und/oder einen optischen, kapazitiven und/oder induktiven Sensor auf.

Vorzugsweise sind der Teilzähler 23 und der Gesamtzähler 25 derart ausgebildet, dass mit ihnen dieselben Benutzungen erfassbar sind bzw. erfasst werden. So ist eine doppelte bzw. mehrfache Zählung derselben Benutzungen ermöglicht, insbesondere wobei die erfassten Benutzungen von dem Teilzähler 23 und dem Gesamtzähler 25 jedoch auf unterschiedliche Weise und/oder getrennt und/oder unabhängig voneinander angezeigt, gespeichert und/oder weiterverarbeitet werden.

Vorzugsweise werden die von dem Teilzähler 23 und dem Gesamtzähler 25 erfassten Benutzungen von derselben Detektionseinrichtung 28 detektiert. Es kann also beispielsweise vorgesehen sein, dass, wenn von der Detektionseinrichtung 28 eine Benutzung detektiert wurde, eine diesbezügliche Information sowohl an den Teilzähler 23 als auch an den Gesamtzähler 25 übertragen oder weitergeleitet wird, insbesondere elektronisch und/oder mechanisch, beispielsweise über das Getriebe. Alternativ oder zusätzlich kann die Information jedoch auch zunächst nur an einen der beiden Zähler 23, 25 übertragen oder weitergeleitet werden, wobei dann im Anschluss der eine der beiden Zähler 23, 25 die Information an den anderen der beiden Zähler 23, 25 weitergibt.

Der Gesamtzähler 25 weist vorzugsweise eine Anzeigeeinrichtung 32 auf. Mit der Anzeigeeinrichtung 32 sind vorzugsweise die bereits (mit dem Behälter 3) erfolgten und/oder noch (mit dem Behälter 3) möglichen Benutzungen anzeigbar. Die Anzeigeeinrichtung 32 kann eine mechanische und/oder eine elektronische Anzeigeeinrichtung 32 sein.

Vorzugsweise weist die Anzeigeeinrichtung 32 ein Anzeigeteil, beispielsweise in Form eines Schiebers, auf, das auf einer Gewindestange angeordnet ist, wobei die Gewindestange insbesondere parallel zur Drehachse D verläuft. Vorzugsweise wird bei einer Benutzung des Zerstäubers 1 das Anzeigeteil axial auf der Gewindestange verschoben, so dass die axiale Position des Anzeigeteils zu der Anzahl der bereits erfolgten Benutzungen korrespondiert.

Der Gesamtzähler 25 und/oder die Anzeigeeinrichtung 32 sind vorzugsweise innerhalb des Gehäuses des Zerstäubers 1 angeordnet. Besonders bevorzugt sind der Gesamtzähler 25 und/oder die Anzeigeeinrichtung 32 innerhalb des Gehäuseunterteils 18 angeordnet. Dabei ist das Gehäuseunterteil 18 vorzugsweise zumindest abschnittsweise durchsichtig ausgebildet, so dass die Anzeigeeinrichtung 32 durch das Gehäuseunterteil 18 hindurch von einem nicht dargestellten Benutzer ablesbar ist.

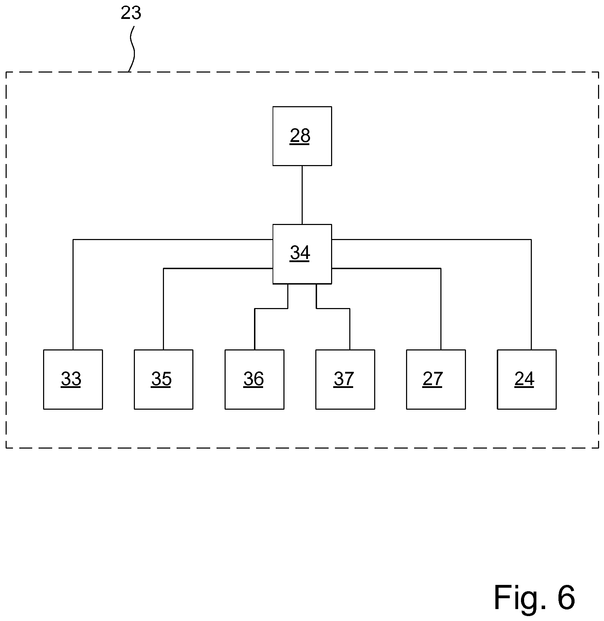

Im Folgenden wird vorrangig auf eine elektronische Ausführungsform des Teilzählers 23 eingegangen, wobei in Fig. 6 einige Merkmale des Teilzählers 23 schematisch dargestellt sind.

Insbesondere kann bei einer elektronischen Ausführungsform des Teilzählers 23 - abweichend von dem in Zusammenhang mit Fig. 5 erläuterten Beispiel - auch die Detektionseinrichtung 28 elektronisch statt mechanisch ausgebildet sein. Alternativ oder zusätzlich ist es denkbar, dass die Detektionseinrichtung 28 eines elektronischen Teilzählers 23 dazu ausgebildet ist, Signale von einem ebenfalls elektronisch ausgebildeten Gesamtzähler 25 zu empfangen.

Der Teilzähler 23 kann dazu ausgebildet sein, eine Anzahl von innerhalb eines dem Teilzähler 23 vorgegebenen oder vorgebbaren Zeitfensters erfolgten Benutzungen des Zerstäubers 1 zu ermitteln. Die Informationen über die innerhalb des Zeitfensters erfolgten und/oder zu erfolgenden Benutzungen sind vorzugsweise mittels des Teilzählers 23 bzw. der Anzeigeeinrichtung 27 ausgebbar oder anzeigbar.

Die angezeigten bzw. ausgegebenen Informationen können beispielsweise die Anzahl der innerhalb des Zeitfensters erfolgten Benutzungen und/oder die innerhalb des Zeitfensters zu erfolgenden Benutzungen oder einen, den nächsten oder die Zeitpunkte der Benutzungen umfassen.

Der Teilzähler 23 weist vorzugsweise eine Benutzungsvorgabeeinrichtung auf. Vorzugsweise ist der Teilzähler 23 mittels der Benutzungsvorgabeeinrichtung gemäß einer Benutzungsvorgabe eingestellt oder einstellbar. Der Begriff "Benutzungsvorgabe" wurde bereits eingangs erläutert.

Die Benutzungsvorgabeeinrichtung kann beispielsweise ein mechanisches Bauteil oder ein Abschnitt eines mechanischen Bauteils des Teilzählers 23 sein, etwa in Form eines austauschbaren Zählrings und/oder in Form einer Sperre. Insbesondere kann die Benutzungsvorgabeeinrichtung dazu ausgebildet sein, den Zerstäuber 1 gegen eine (weitere) Benutzung zu blockieren bzw. zu sperren, sodass nach einer bestimmten bzw. vordefinierten Anzahl von Benutzungen gemäß der Benutzungsvorgabe keine weitere Benutzung des Zerstäubers 1 möglich ist.

Es ist insbesondere auch möglich, dass die Benutzungsvorgabeeinrichtung gemäß der Benutzungsvorgabe einstellbar ist oder dass die Benutzungsvorgabeeinrichtung austauschbar ist, sodass durch Austauschen der Benutzungsvorgabeeinrichtung der Teilzähler 23 bzw. Zerstäuber 1 gemäß unterschiedlicher Benutzungsvorgaben einstellbar ist bzw. an unterschiedliche Benutzungsvorgaben anpassbar ist.

Der Teilzähler 23 weist vorzugsweise eine Kommunikationseinrichtung 33 auf, die zur drahtgebundenen und/oder bevorzugt drahtlosen Kommunikation ausgebildet ist. Insbesondere kann die Kommunikation über WLAN, Bluetooth, Infrarot, NFC, RFID und/oder sonstige geeignete Standards erfolgen.

Die Kommunikationseinrichtung 33 sowie weitere im Folgenden erläuterte Merkmale des Teilzählers 23 sind in Fig. 5 schematisch dargestellt.

Mittels der Kommunikationseinrichtung 33 kann der Teilzähler 23 beispielsweise mit dem Zerstäuber 1 bzw. einem anderen Teil des Zerstäubers 1 und/oder mit einem externen System kommunizieren bzw. Daten austauschen. Weiter kann das externe System beispielsweise ein Computer, insbesondere in Form eines PCs, Servers oder eines tragbaren Geräts wie eines Smartphones, Tablets oder Laptops, sein. Das externe System kann jedoch auch ein Netzwerk wie etwa das Internet sein, sodass die Informationen über dieses Netzwerk direkt an einen Arzt oder sonstigen medizinischen Betreuer des Benutzers des Zerstäubers 1 übermittelbar sind.

Auf diese Weise kann der Teilzähler 23 als Compliance-Schnittstelle ausgebildet sein und/oder zur Patientenüberwachung bzw. Therapieüberwachung dienen. Der Teilzähler 23 kann also mit anderen Worten dazu ausgebildet sein, die Compliance bzw. Therapietreue eines Benutzers bzw. Patienten zu verbessern und/oder zu kontrollieren. Vorzugsweise sind die Benutzungen des Zerstäubers 1 so insbesondere aufzeichenbar, verfolgbar und/oder kontrollierbar, sodass insbesondere eine Therapietreue bzw. ein Befolgen der Benutzungsvorgabe verfolgbar oder überprüfbar ist. Der Teilzähler 23 ist vorzugsweise dazu ausgebildet, Daten, die mit der Compliance bzw. Therapietreue in Zusammenhang stehen bzw. diese betreffen, zu senden und/oder zu empfangen

In einer bevorzugten Ausführungsform ist die Benutzungsvorgabeeinrichtung elektronisch und/oder als Software bzw. Softwaremodul, wie bereits eingangs erläutert. Insbesondere kann die Benutzungsvorgabeeinrichtung als Programm bzw. Software(modul) ausgebildet sein, wobei mittels des Programms bzw. der Software eine Einstellung, Änderung und/oder Überprüfung der Benutzungsvorgabe durchführbar ist.

Vorzugsweise ist der Teilzähler 23 mittels der Benutzungsvorgabeeinrichtung dazu ausgebildet, die erfolgten und/oder noch zu erfolgenden Benutzungen mit der Benutzungsvorgabe abzugleichen bzw. zu vergleichen.

Insbesondere weist der Teilzähler 23 eine CPU bzw. einen Prozessor 34 und einen Speicher 35 auf. Vorzugsweise ist die Benutzungsvorgabe in dem Speicher 35 gespeichert oder speicherbar. Der Prozessor 34 ist vorzugsweise zum Zugreifen auf den Speicher 35 und/oder zum Lesen, Schreiben und/oder Löschen des Speichers 35 ausgebildet.

Die Benutzungsvorgabe ist vorzugsweise in dem Speicher 35 abgelegt oder gespeichert, insbesondere in Form einer Tabelle, Liste, Matrix und/oder sonstigen geeigneten Datenstruktur.

Die Benutzungen sind mit der Benutzungsvorgabe vorzugsweise automatisch abgleichbar, insbesondere wobei der Prozessor 34 zum Abgleichen der Benutzungen mit der Benutzungsvorgabe ausgebildet ist.

Insbesondere kann der Teilzähler 23 und/oder Prozessor 34 dazu ausgebildet sein, Zeitpunkte von Benutzungen und/oder zeitliche Abstände zwischen Benutzungen zu ermitteln. Vorzugsweise weist der Teilzähler 23 dazu einen Zeitgeber 36 auf.

Mittels des Zeitgebers 36 sind vorzugsweise die Zeitpunkte der Benutzungen zumindest gemäß einer relativen bzw. zeitgeber-internen Zeit feststellbar. Insbesondere können auch, beispielsweise mittels des Prozessors 34, zeitliche Abstände zwischen verschiedenen Benutzungen ermittelt werden.

Es ist auch möglich, dass lediglich feststellbar ist, ob der zeitliche Abstand zwischen zwei (detektierten) Benutzungen größer oder kleiner als ein Referenzwert ist. So kann beispielsweise festgestellt werden, ob zwei oder mehr Benutzungen des Zerstäubers 1 direkt hintereinander erfolgt sind.

Der Prozessor 34 ist vorzugsweise mit der Detektionseinrichtung 28 (informationstechnisch) verbunden bzw. gekoppelt, wodurch eine Information oder ein Messergebnis über eine erfolgte Benutzung von der Detektionseinrichtung 28 an den Prozessor 34 übertragbar ist. Vorzugsweise werden die Anzahl, die Zeitpunkte und/oder die zeitlichen Abstände der erfolgten Benutzungen von dem Prozessor 34 in dem Speicher 35 gespeichert bzw. abgelegt. Weiter ist der Prozessor 34 dazu ausgebildet, die Anzahl, die Zeitpunkte und/oder die zeitlichen Abstände der erfolgten Benutzungen mit der Benutzungsvorgabe, insbesondere automatisch, abzugleichen.

Die Benutzungsvorgabe kann insbesondere einen Zeitplan aufweisen, in dem relative und/oder absolute Zeitpunkte für Benutzungen eingetragen bzw. vorgegeben sind und/oder in dem zeitliche Abstände, insbesondere Mindest- oder Maximalabstände, zwischen Benutzungen vorgegeben oder festgelegt sind. So kann eine Therapie optimiert werden.

Vorzugsweise ist der Prozessor 34 dazu ausgebildet, die Benutzungen automatisch mit der Benutzungsvorgabe abzugleichen bzw. gleicht der Prozessor 34 die Benutzungen automatisch mit der Benutzungsvorgabe ab.

Insbesondere sind mit der Kommunikationseinrichtung 33 Informationen über erfolgte und/oder zu erfolgende Benutzungen sendbar und/oder empfangbar.

Besonders bevorzugt ist mittels der Kommunikationseinrichtung 33 ein Zählerwert bzw. Zählerstand des Teilzählers 23 und/oder des Gesamtzählers 25 sendbar oder abrufbar.

Vorzugsweise ist die Benutzungsvorgabe bzw. sind Informationen über die Benutzungsvorgabe und/oder ein Ergebnis eines Abgleichs der Benutzungsvorgabe mit detektierten bzw. erfolgten oder gezählten Benutzungen mit der Kommunikationseinrichtung 33 sendbar und/oder empfangbar.

Die Informationen über die Benutzungen können des Weiteren Zeitpunkte von und/oder zeitliche Abstände zwischen Benutzungen und/oder Auslösungen des Zerstäubers 1 umfassen (Sollwerte und/oder Ergebnisse einer Detektion).

Vorzugsweise sind die Benutzungsvorgabe und/oder das Ergebnis des Abgleichs mit der Benutzungsvorgabe mittels des Teilzählers 23 bzw. der Anzeigeeinrichtung 27 ausgebbar oder anzeigbar.

Der Teilzähler 23 weist vorzugsweise einen Signalgeber 37 auf. Insbesondere ist der Signalgeber 37 dazu ausgebildet, in Abhängigkeit von dem Ergebnis des Abgleichs der Benutzungen mit der Benutzungsvorgabe ein Signal auszugeben.

Der Signalgeber 37 kann als optischer und/oder akustischer Signalgeber 37 ausgebildet sein.

Es ist möglich, dass die Anzeigeeinrichtung 27 den Signalgeber 37 aufweist oder bildet. Der Signalgeber 37 kann jedoch alternativ oder zusätzlich einen Lautsprecher und/oder ein oder mehrere Leuchtmittel, z.B. LEDs, aufweisen oder dadurch gebildet sein.

Insbesondere kann einem nicht dargestellten Benutzer mittels des Signalgebers 37 das Ergebnis des Abgleichs der Benutzungsvorgabe mit den Benutzungen optisch und/oder akustisch mitgeteilt werden.

Besonders bevorzugt erfolgt die Ausgabe eines Signals, wenn der Abgleich ergeben hat, dass die Benutzung des Zerstäubers 1 nicht mit der Benutzungsvorgabe übereinstimmt bzw. von dieser abweicht, insbesondere dass eine durch die Benutzungsvorgabe vorgegebene Benutzung nicht erfolgt ist. Das in diesem Fall ausgegebene Signal kann z. B. ein Piepton, eine angezeigte Textinformation, ein Blinken und/oder ein sonstiges optisches und/oder akustisches (Erinnerungs- bzw. Warn-) Signal sein.

Es ist jedoch auch möglich, dass die Ausgabe eines Signals ohne einen Abgleich der Benutzungsvorgabe mit den Benutzungen erfolgt. Beispielsweise kann auch ein optisches und/oder akustisches Signal ausgegeben werden, wenn durch den Teilzähler 23 eine Benutzung des Zerstäubers 1 ermittelt wurde, sodass dieses Signal dem Benutzer als Bestätigung einer (korrekten) Benutzung bzw. einer der Benutzungsvorgabe entsprechenden Benutzung des Zerstäubers 1 dient. So kann eine korrekte Bedienung bzw. Benutzung des Zerstäubers 1 unterstützt werden.

Einem Patienten bzw. Benutzer des Zerstäubers 1 wird auf diese Weise das Einhalten bzw. Befolgen des Therapieplans bzw. der Benutzungsvorgabe erleichtert. Insbesondere kann der Benutzer so an versäumte Benutzungen bzw. Medikamenteneinnahmen erinnert werden und/oder zur Benutzung des Zerstäubers 1 bzw. Einnahme des Medikaments aufgefordert werden. Die Compliance bzw. Therapietreue des Patienten kann so verbessert oder optimiert werden.

Der Teilzähler 23 kann dazu ausgebildet sein, eine weitere Betätigung bzw. Benutzung des Zerstäubers 1 zu verhindern bzw. zu blockieren. Insbesondere kann dies in Abhängigkeit von bereits erfolgten Benutzungen, in Abhängigkeit vom Zählerstand oder Zählerwert und/oder in Abhängigkeit des Ergebnisses des Abgleichs von erfolgten Benutzungen mit der Benutzungsvorgabe erfolgen. Eine Blockade erfolgt vorzugsweise dadurch, dass ein Verdrehen der Gehäuseteile 16, 17, 18 gegeneinander insbesondere mechanisch blockiert wird.

Gemäß einem weiteren, auch unabhängig realisierbaren Aspekt kann der Zerstäuber 1 mit einer dem Zerstäuber 1 zugeordneten Dockingstation 38 ein System bilden. Dies ist in Fig. 3 angedeutet.

Die Dockingstation 38 ist vorzugsweise zum Laden eines (elektrischen) Energiespeichers des Teilzählers 23 und/oder Zerstäubers 1 und/oder zur Kommunikation mit dem Teilzähler 23 und/oder Zerstäuber 1 ausgebildet.

Die Dockingstation 38 ist vorzugsweise dazu ausgebildet, dass der Zerstäuber 1 in die Dockingstation 38 einsetzbar und/oder durch Einsetzen in die Dockingstation 38 mit der Dockingstation 38 verbindbar bzw. koppelbar ist.

Der Zerstäuber 1 kann aber auch auf andere Weise als durch Einsetzen mit der Dockingstation 38 koppelbar sein. Beispielsweise kann die Dockingstation 38 als induktive Ladeplatte o. dgl. ausgebildet sein, so dass zum Koppeln des Zerstäubers 1 mit der Ladeplatte bzw. Dockingstation 38 bereits ein Auflegen des Zerstäubers 1 auf die Ladeplatte bzw. Dockingstation 38 genügt.

Vorzugsweise ist die Dockingstation 38 dazu ausgebildet, das Einsetzen bzw. Koppeln des Zerstäubers 1 zu detektieren.

Vorzugsweise weist die Dockingstation 38 eine Kommunikationseinrichtung 39 auf, insbesondere wobei die Kommunikationseinrichtung 39 der Dockingstation 38 zur bevorzugt drahtlosen Kommunikation mit der Kommunikationseinrichtung 33 des Teilzählers 23 ausgebildet ist.

Vorzugsweise sind mittels bzw. durch die Kommunikationseinrichtungen 33, 39 Informationen über erfolgte und/oder zu erfolgende Benutzungen zwischen dem Zerstäuber 1 und der Dockingstation 38 austauschbar.

Ein Laden eines Energiespeichers des Teilzählers 23 und/oder des Zerstäubers 1 durch die Dockingstation 38 kann insbesondere in Abhängigkeit der Informationen über erfolgte und/oder zu erfolgende Benutzungen erfolgen.

Alternativ oder zusätzlich kann die Dockingstation 38 auch dazu ausgebildet sein, den Energiespeicher 7 zu laden bzw. die Feder des Zerstäubers 1 zu spannen. Vorzugsweise ist die Dockingstation 38 bzw. das System mit dem Zerstäuber 1 und der Dockingstation 38 in diesem Fall dazu ausgebildet, dass ein Laden des Energiespeichers 7 bzw. ein Spannen der Feder mittels der Dockingstation 38 in Abhängigkeit von dem Ergebnis eines Abgleichs der Benutzungsvorgabe mit tatsächlich erfolgten Benutzungen erfolgt.

### Bezugszeichenliste:

- 1: Zerstäuber
- 2: Fluid
- 3: Behälter
- 4: Fluidraum
- 5: Druckerzeuger
- 6: Halterung
- 7: Energiespeicher
- 8: Sperrelement
- 8a: Auslösetaste
- 9: Förderrohr
- 10: Rückschlagventil
- 11: Druckkammer
- 12: Austragsdüse
- 13: Mundstück
- 14: Aerosol
- 15: Zuluftöffnung
- 16: (oberes) Gehäuseteil
- 17: Innenteil
- 17a: oberer Teil (Innenteil)
- 17b: unterer Teil (Innenteil)
- 18: (unteres) Gehäuseteil
- 19: Halteelement
- 20: Feder
- 21: Behälterboden
- 22: Anstechelement
- 23: Teilzähler
- 24: Rücksetzeinrichtung
- 25: Gesamtzähler
- 26: Betätigungselement
- 27: Anzeigeeinrichtung (Teilzähler)
- 28: Detektionseinrichtung
- 29: erstes Kopplungselement
- 30: zweites Kopplungselement
- 31: Anzeigeelement (Teilzähler)
- 32: Anzeigeeinrichtung (Gesamtzähler)
- 33: Kommunikationseinrichtung
- 34: Prozessor
- 35: Speicher
- 36: Zeitgeber
- 37: Signalgeber
- 38: Dockingstation
- 39: Kommunikationseinrichtung

- D: Drehachse

## Patentansprüche

1. Teilzähler (23) zum Zählen eines Teils von Benutzungen eines Zerstäubers (1) zur Erzeugung eines einatembaren Aerosols (14), wobei der Teilzähler (23) mit dem Zerstäuber (1) koppelbar und von dem Zerstäuber (1) abnehmbar ist, wobei der Teilzähler (23) eine Detektionseinrichtung (28) zum Detektieren von Benutzungen des Zerstäubers (1) und eine Rücksetzeinrichtung (24) zum Zurücksetzen des Teilzählers (23) aufweist,
**dadurch gekennzeichnet,**
**dass** die Detektionseinrichtung (28) zwei Kopplungselemente (29, 30) aufweist, die zur mechanischen Kopplung mit unterschiedlichen Gehäuseteilen (16, 17, 18) des Zerstäubers (1) ausgebildet sind, sodass eine Drehung der Gehäuseteile (16, 17, 18) gegeneinander mittels der Kopplungselemente (29, 30) derart auf den Teilzähler (23) übertragbar ist, dass dieser antreibbar bzw. triggerbar ist, wobei die Detektionseinrichtung (28) dazu ausgebildet ist, eine Drehung der Gehäuseteile (16, 17, 18) gegeneinander zu detektieren und als Benutzung zu zählen.

2. Teilzähler nach Anspruch 1, **dadurch gekennzeichnet, dass** der Teilzähler (23) zur Ermittlung einer Anzahl von innerhalb eines dem Teilzähler (23) vorgegebenen oder vorgebbaren Zeitfensters erfolgten Benutzungen des Zerstäubers (1) und zur Ausgabe von Informationen über die innerhalb des Zeitfensters erfolgten und/oder zu erfolgenden Benutzungen ausgebildet ist.

3. Teilzähler nach Anspruch 1 oder 2, **dadurch gekennzeichnet, dass** der Teilzähler (23) - insbesondere durch Aufschieben - mit einem Gehäuseteil (16, 17, 18) des Zerstäubers (1) verbindbar ist, vorzugsweise wobei der Teilzähler (23) mit dem Zerstäuber (1) durch Aufschieben derart koppelbar ist, dass Benutzungen des Zerstäubers (1) mit der Detektionseinrichtung (28) detektierbar sind.

4. Teilzähler nach einem der voranstehenden Ansprüche, **dadurch gekennzeichnet, dass** die Detektionseinrichtung (28) dazu ausgebildet ist, Auslösevorgänge oder Ladevorgänge als Benutzung des Zerstäubers (1) zu erfassen oder zu zählen.

5. Teilzähler nach einem der voranstehenden Ansprüche, **dadurch gekennzeichnet, dass** der Teilzähler (23) dazu ausgebildet ist, in einer Gebrauchslage, in der Benutzungen des Zerstäubers (1) durch den Teilzähler (23) ermittelbar sind, ein Gehäuseteil (16, 17, 18) des Zerstäubers (1) zumindest teilweise zu umgreifen.

6. Teilzähler nach einem der voranstehenden Ansprüche, **dadurch gekennzeichnet, dass** der Teilzähler (23) ein Betätigungselement (26) aufweist, wobei durch Betätigung des Betätigungselements (26) der Teilzähler (23) zurücksetzbar ist, und/oder dass der Teilzähler (23) eine elektronische Anzeigeeinrichtung (27) zur optischen Anzeige der Informationen über die Benutzungen aufweist.

7. Teilzähler nach einem der voranstehenden Ansprüche, **dadurch gekennzeichnet, dass** der Teilzähler (23) eine Kommunikationseinrichtung (28) zur drahtlosen Kommunikation aufweist und dazu ausgebildet ist, mit der Kommunikationseinrichtung (28) Informationen über erfolgte und/oder zu erfolgende Benutzungen zu senden und/oder zu empfangen.

8. Teilzähler nach einem der voranstehenden Ansprüche, **dadurch gekennzeichnet, dass** der Teilzähler (23) eine Benutzungsvorgabeeinrichtung aufweist und dazu ausgebildet ist, die erfolgten oder zu erfolgenden Benutzungen mit der Benutzungsvorgabe automatisch abzugleichen, vorzugsweise wobei der Teilzähler (23) einen Signalgeber (37) aufweist, der dazu ausgebildet ist, in Abhängigkeit von dem Ergebnis des Abgleichs von erfolgten oder zu erfolgenden Benutzungen mit der Benutzungsvorgabe ein Signal auszugeben.

9. Teilzähler nach einem der voranstehenden Ansprüche, **dadurch gekennzeichnet, dass** der Teilzähler (23) dazu ausgebildet ist, Zeitpunkte von oder zeitliche Abstände zwischen Benutzungen zu ermitteln.

10. Zerstäuber (1) zur Erzeugung eines einatembaren Aerosols (14) mit einem Behälter (3) für eine zu zerstäubende Substanz und einem Teilzähler (23) zum Ermitteln von Benutzungen des Zerstäubers (1) nach einem der voranstehenden Ansprüche,
wobei der Zerstäuber (1) zusätzlich zu dem Teilzähler (23) einen Gesamtzähler (25) aufweist, mit dem die insgesamt mit dem Behälter (3) erfolgten Benutzungen erfassbar sind, vorzugsweise wobei die Anzahl der insgesamt mit dem Behälter erfolgten Benutzungen der Anzahl der insgesamt mit dem Behälter abgegebenen Dosen des Aerosols (14) entspricht; und/oder
wobei der Teilzähler (23) zur Ermittlung einer Anzahl von innerhalb eines dem Teilzähler (23) vorgegebenen oder vorgebbaren Zeitfensters erfolgten Benutzungen des Zerstäubers (1) und zur Ausgabe von Informationen über die innerhalb des Zeitfensters erfolgten und/oder zu erfolgenden Benutzungen ausgebildet ist.

11. Zerstäuber nach Anspruch 10, **dadurch gekennzeichnet, dass** der Teilzähler (23) an einer Grenze zwischen zwei Gehäuseteilen (16, 17, 18) des Zerstäubers (1) anordenbar ist, wobei der Teilzähler (23) mit beiden Gehäuseteilen (16, 17, 18) in Kontakt bringbar ist, vorzugsweise so dass mit dem Teilzähler (23) eine relative Bewegung der Gehäuseteile (16, 17, 18) zueinander, insbesondere eine Drehung der Gehäuseteile (16, 17, 18) gegeneinander, detektierbar oder als Benutzung des Zerstäubers (1) zählbar ist.

12. Zerstäuber nach Anspruch 10 oder 11, **dadurch gekennzeichnet, dass** der Zerstäuber (1) zusätzlich zu dem Teilzähler (23) einen Gesamtzähler (25) aufweist, vorzugsweise wobei der Teilzähler (23) und der Gesamtzähler (25) dazu ausgebildet sind, dieselben Benutzungen zu erfassen, wobei mit dem Gesamtzähler (25) die insgesamt mit dem Behälter (3) erfolgten Benutzungen erfassbar sind, vorzugsweise wobei die Anzahl der insgesamt mit dem Behälter (3) erfolgten Benutzungen der Anzahl der insgesamt mit dem Behälter (3) abgegebenen Dosen des Aerosols (14) entspricht.

13. Zerstäuber nach einem der Ansprüche 10 bis 12, **dadurch gekennzeichnet, dass** der Zerstäuber (1) einen Druckerzeuger (5) aufweist, wobei mittels eines Energiespeichers (7) des Druckerzeugers (5) die zu zerstäubende Substanz unter Druck setzbar und über eine Austragsdüse (12) als vorzugsweise einatembares Aerosol (14) ausgebbar ist, vorzugsweise wobei der Teilzähler (23) dazu ausgebildet ist, ein Verdrehen von Gehäuseteilen (16, 17, 18) gegeneinander, eine axiale Position des Behälters (3), eine Bewegung des Behälters (3), ein Laden eines Energiespeichers (7), ein Spannen einer Feder, eine Betätigung einer Auslösetaste (8a), einen Austrag des Aerosols (14) oder ein Einatmen des Aerosols (14) als Benutzung zu erfassen.

14. System mit einem Zerstäuber (1) nach einem der Ansprüche 10 bis 13 und einer Dockingstation (38), wobei die Dockingstation (38) zum Laden eines Energiespeichers des Teilzählers (23) oder des Zerstäubers (1) und/oder zur Kommunikation mit dem Teilzähler (23) oder dem Zerstäuber (1) ausgebildet ist, vorzugsweise wobei der Zerstäuber (1) und die Dockingstation (38) jeweils eine Kommunikationseinrichtung (33, 39) zur drahtlosen Kommunikation aufweisen, sodass durch die Kommunikationseinrichtungen (33, 39) Informationen über erfolgte oder zu erfolgende Benutzungen zwischen dem Zerstäuber (1) und der Spannvorrichtung austauschbar sind.

## Claims

1. A sub-counter (23) for counting a share of uses of an atomizer (1) for producing an inhalable aerosol (14), the sub-counter (23) being couplable to the atomizer (1) and being detachable from the atomizer (1), the sub-counter (23) comprising a detection device (28) for detecting uses of the atomizer (1) and a resetting means (24) for resetting the sub-counter (23),
**characterised in that**
**in that** the detection device (28) has two coupling elements (29, 30) which are configured for mechanical coupling to different housing parts (16, 17, 18) of the atomizer (1), so that a rotation of the housing parts (16, 17, 18) with respect to one another is transmittable by means of the coupling elements (29, 30) to the sub-counter (23) in such a way that the latter can be driven or triggered, wherein the detection device (28) is configured to detect a rotation of the housing parts (16, 17, 18) with respect to one another and to count it as use.

2. Sub-counter according to claim 1, **characterized in that** the sub-counter (23) is configured to determine a number of uses of the atomizer (1) which have taken place within a time window which is predetermined or can be predetermined for the sub-counter (23) and to output information about the uses which have taken place and/or are to take place within the time window.

3. Sub-counter according to claim 1 or 2, **characterized in that** the sub-counter (23) can be connected - in particular by sliding on - to a housing part (16, 17, 18) of the atomizer (1), preferably wherein the sub-counter (23) can be coupled to the atomizer (1) by sliding on in such a way that uses of the atomizer (1) can be detected with the detection device (28).

4. Sub-counter according to any one of the preceding claims, **characterized in that** the detection device (28) is configured to detect or count triggering or charging operations as use of the atomizer (1).

5. Sub-counter according to one of the preceding claims, **characterized in that** the sub-counter (23) is configured to at least partially surround a housing part (16, 17, 18) of the atomizer (1) in a position of use in which uses of the atomizer (1) can be determined by the sub-counter (23).

6. Sub-counter according to one of the preceding claims, **characterized in that** the sub-counter (23) has an actuating element (26), wherein the sub-counter (23) can be reset by actuating the actuating element (26), and/or **in that** the sub-counter (23) has an electronic display device (27) for optically displaying the information about the uses.

7. Sub-counter according to one of the preceding claims, **characterized in that** the sub-counter (23) comprises a communication device (28) for wireless communication and is designed to send and/or receive information with the communication device (28) about uses that have taken place and/or are to take place.

8. Sub-counter according to one of the preceding claims, **characterized in that** the sub-counter (23) has a usage specification device and is configured to automatically compare the uses which have occurred or are to occur with the usage specification, preferably wherein the sub-counter (23) has a signal transmitter (37) which is configured to output a signal depending on the result of the comparison of uses which have occurred or are to occur with the usage specification.

9. Sub-counter according to one of the preceding claims, **characterized in that** the sub-counter (23) is configured to determine times of or time intervals between uses.

10. Atomizer (1) for producing an inhalable aerosol (14), comprising a container (3) for a substance to be atomized and a sub-counter (23) for detecting uses of the atomizer (1) according to any one of the preceding claims,
wherein the nebulizer (1) comprises, in addition to the sub-counter (23), a total counter (25) by means of which the total number of uses made with the container (3) can be detected, preferably wherein the total number of uses made with the container corresponds to the total number of doses of aerosol (14) dispensed with the container; and/or
wherein the sub-counter (23) is configured to determine a number of uses of the atomizer (1) which have taken place within a time window which is predetermined or can be predetermined for the sub-counter (23) and to output information about the uses which have taken place and/or are to take place within the time window.

11. Atomizer according to claim 10, **characterized in that** the sub-counter (23) is arrangeable at a boundary between two housing parts (16, 17, 18) of the atomizer (1), wherein the sub-counter (23) can be brought into contact with both housing parts (16, 17, 18), preferably in such a way that a relative movement of the housing parts (16, 17, 18) with respect to one another, in particular a rotation of the housing parts (16, 17, 18) with respect to one another, can be detected or counted as use of the atomizer (1) with the sub-counter (23).

12. Atomizer according to claim 10 or 11, **characterized in that** the atomizer (1) has a total counter (25) in addition to the sub-counter (23), preferably wherein the sub-counter (23) and the total counter (25) are configured to detect the same uses, wherein the total counter (25) is adapted to detect the total number of uses made with the container (3), preferably wherein the total number of uses made with the container (3) corresponds to the total number of doses of the aerosol (14) dispensed with the container (3).

13. Atomizer according to one of the claims 10 to 12, **characterized in that** the atomizer (1) has a pressure generator (5), wherein the substance to be atomized can be pressurized by means of an energy storage (7) of the pressure generator (5) and can be discharged via a discharge nozzle (12) as a preferably inhalable aerosol (14), preferably wherein the sub-counter (23) is configured to detect twisting of housing parts (16, 17, 18) relative to one another, an axial position of the container (3), a movement of the container (3), a charging of an energy storage (7), a tensioning of a spring, an actuation of a release button (8a), a discharge of the aerosol (14), or an inhalation of the aerosol (14) as use.

14. System comprising an atomizer (1) according to any one of claims 10 to 13 and a docking station (38), wherein the docking station (38) is configured for charging an energy storage of the sub-counter (23) or the atomizer (1) and/or for communication with the sub-counter (23) or the atomizer (1), preferably wherein the atomizer (1) and the docking station (38) each comprise a communication device (33, 39) for wireless communication, so that by means of the communication devices (33, 39) information about uses made or to be made can be exchanged between the atomizer (1) and the tensioning device.

## Revendications

1. Compteur partiel (23) destiné à compter une partie d'utilisations d'un pulvérisateur (1) destiné à produire un aérosol (14) inhalable, dans lequel le compteur partiel (23) peut être couplé au pulvérisateur (1) et peut être retiré du pulvérisateur (1), dans lequel le compteur partiel (23) présente un dispositif de détection (28) destiné à détecter des utilisations du pulvérisateur (1) et un dispositif de réinitialisation (24) destiné à réinitialiser le compteur partiel (23),
**caractérisé en ce**
**que** le dispositif de détection (28) présente deux éléments de couplage (29, 30), qui sont réalisés pour être couplés mécaniquement à différentes parties de boîtier (16, 17, 18) du pulvérisateur (1) si bien qu'une rotation des parties de boîtier (16, 17, 18) les unes à l'encontre des autres peut être transmise au moyen des éléments de couplage (29, 30) de telle manière sur le compteur partiel (23) que ce dernier peut être entraîné ou déclenché, dans lequel le dispositif de détection (28) est réalisé pour détecter une rotation des parties de boîtier (16, 17, 18) les unes à l'encontre des autres et pour la compter en tant qu'utilisation.

2. Compteur partiel selon la revendication 1, **caractérisé en ce que** le compteur partiel (23) est réalisé pour déterminer un nombre d'utilisations du pulvérisateur (1) ayant eu lieu dans une fenêtre temporelle spécifiée ou pouvant être spécifiée au compteur partiel (23) et pour délivrer des informations sur les utilisations ayant eu lieu et/ou devant avoir lieu dans la fenêtre temporelle.

3. Compteur partiel selon la revendication 1 ou 2, **caractérisé en ce que** le compteur partiel (23) peut être relié - en particulier par emboîtement - à une partie de boîtier (16, 17, 18) du pulvérisateur (1), de préférence dans lequel le compteur partiel (23) peut être couplé au pulvérisateur (1) par emboîtement de telle manière que des utilisations du pulvérisateur (1) peuvent être détectées avec le dispositif de détection (28).

4. Compteur partiel selon l'une quelconque des revendications précédentes, **caractérisé en ce que** le dispositif de détection (28) est réalisé pour détecter ou pour compter des opérations de déclenchement ou des opérations de chargement en tant qu'utilisation du pulvérisateur (1).

5. Compteur partiel selon l'une quelconque des revendications précédentes, **caractérisé en ce que** le compteur partiel (23) est réalisé pour entourer au moins en partie une partie de boîtier (16, 17, 18) du pulvérisateur (1) dans une position d'utilisation, dans laquelle des utilisations du pulvérisateur (1) peuvent être déterminées par le compteur partiel (23).

6. Compteur partiel selon l'une quelconque des revendications précédentes, **caractérisé en ce que** le compteur partiel (23) présente un élément d'actionnement (26), dans lequel le compteur partiel (23) peut être réinitialisé par l'actionnement de l'élément d'actionnement (26), et/ou que le compteur partiel (23) présente un dispositif d'affichage électronique (27) destiné à afficher de manière optique les informations sur les utilisations.

7. Compteur partiel selon l'une quelconque des revendications précédentes, **caractérisé en ce que** le compteur partiel (23) présente un dispositif de communication (28) pour la communication sans fil et est réalisé pour envoyer et/ou recevoir avec le dispositif de communication (28) des informations sur les utilisations ayant eu lieu et/ou devant avoir lieu.

8. Compteur partiel selon l'une quelconque des revendications précédentes, **caractérisé en ce que** le compteur partiel (23) présente un dispositif de spécification d'utilisation et est réalisé pour comparer de manière automatique les utilisations ayant eu lieu ou devant avoir lieu à la spécification d'utilisation, dans lequel de préférence le compteur partiel (23) présente un générateur de signaux (37), qui est réalisé pour délivrer un signal en fonction du résultat de la comparaison d'utilisations ayant eu lieu ou devant avoir lieu à la spécification d'utilisation.

9. Compteur partiel selon l'une quelconque des revendications précédentes, **caractérisé en ce que** le compteur partiel (23) est réalisé pour déterminer des moments ou des intervalles de temps entre des utilisations.

10. Pulvérisateur (1) destiné à produire un aérosol inhalable (14) avec un contenant (3) pour une substance à pulvériser et un compteur partiel (23) destiné à déterminer des utilisations du pulvérisateur (1) selon l'une quelconque des revendications précédentes,
dans lequel le pulvérisateur (1) présente, en plus du compteur partiel (23), un compteur total (25), avec lequel les utilisations ayant eu lieu au total avec le contenant (3) peuvent être détectées, dans lequel de préférence le nombre des utilisations ayant eu lieu au total avec le contenant correspond au nombre des doses de l'aérosol (14) distribuées au total avec le contenant ; et/ou
dans lequel le compteur partiel (23) est réalisé pour déterminer un nombre d'utilisations du pulvérisateur (1) ayant eu lieu dans une fenêtre temporelle spécifiée ou pouvant être spécifiée au compteur partiel (23) et pour délivrer des informations sur les utilisations ayant eu lieu et/ou devant avoir lieu dans la fenêtre temporelle.

11. Pulvérisateur selon la revendication 10, **caractérisé en ce que** le compteur partiel (23) peut être disposé sur une limite entre deux parties de compteur (16, 17, 18) du pulvérisateur (1), dans lequel le compteur partiel (23) peut être amené en contact avec deux parties de boîtier (16, 17, 18), de préférence de telle sorte qu'un déplacement relatif des parties de boîtier (16, 17, 18) les unes par rapport aux autres, en particulier une rotation des parties de boîtier (16, 17, 18) les unes à l'encontre des autres, peut être détecté ou peut être compté en tant qu'utilisation du pulvérisateur (1) avec le compteur partiel (23).

12. Pulvérisateur selon la revendication 10 ou 11, **caractérisé en ce que** le pulvérisateur (1) présente un compteur total (25) en plus du compteur partiel (23), dans lequel de préférence le compteur partiel (23) et le compteur total (25) sont réalisés pour détecter les mêmes utilisations, dans lequel les utilisations ayant eu lieu au total avec le contenant (3) peuvent être détectées avec le compteur total (25), dans lequel de préférence le nombre des utilisations ayant eu lieu au total avec le contenant (3) correspond au nombre des doses de l'aérosol (14) distribuées au total avec le contenant (3).

13. Pulvérisateur selon l'une quelconque des revendications 10 à 12, **caractérisé en ce que** le pulvérisateur (1) présente un générateur de pression (5), dans lequel la substance à pulvériser peut être mise sous pression au moyen d'un accumulateur d'énergie (7) du générateur de pression (5) et peut être délivrée en tant qu'aérosol (14) de préférence inhalable par l'intermédiaire d'une buse de décharge (12), dans lequel de préférence le compteur partiel (23) est réalisé pour détecter une rotation de parties de boîtier (16, 17, 18) les unes à l'encontre des autres, une position axiale du contenant (3), un déplacement du contenant (3), un chargement d'un accumulateur d'énergie (7), une mise sous tension d'un ressort, un actionnement d'un bouton de déclenchement (8a), une décharge de l'aérosol (14) ou une inhalation de l'aérosol (14) en tant qu'utilisation.

14. Système avec un pulvérisateur (1) selon l'une quelconque des revendications 10 à 13 et une station d'accueil (38), dans lequel la station d'accueil (38) est réalisée pour charger un accumulateur d'énergie du compteur partiel (23) ou du pulvérisateur (1) et/ou pour communiquer avec le compteur partiel (23) ou le pulvérisateur (1), dans lequel de préférence le pulvérisateur (1) et la station d'accueil (38) présentent respectivement un dispositif de communication (33, 39) pour la communication sans fil si bien que des informations sur des utilisations ayant eu lieu ou devant avoir lieu peuvent être échangées entre le pulvérisateur (1) et le dispositif de mise sous tension par les dispositifs de communication (33, 39).
